# EUROPEAN PATENT SPECIFICATION

(11) **EP 4 157 103 B1**
(45) Date of publication and mention of the grant of the patent: **23.07.2025**
(21) Application number: 21735091.7
(22) Date of filing: 02.06.2021
(51) Int. Cl.: A61B 17/04

(54) **SELF-PUNCHING LATERAL ROW ANCHOR AND ANCHOR DRIVER**
SELBSTSTANZENDER SEITLICHER REIHENANKER UND ANKERTREIBER
ANCRAGE À RANGÉE LATÉRALE AUTO-PERFORANT ET DISPOSITIF D'ENTRAÎNEMENT D'ANCRAGE

(30) Priority: 02.06.2020 US 202063033541 P; 02.06.2020 US 202063033545 P; 11.11.2020 US 202063112389 P
(43) Date of publication of application: 05.04.2023
(73) Proprietor: Conmed Corporation, Largo, FL 33773 (US)
(72) Inventor: PADILLA, Scott, Tampa, FL 33618 (US)
(74) Representative: Strehl & Partner mbB
(86) International application number: PCT/US2021/035450
(87) International publication number: WO 2021/247707

(56) References cited:
- US-A1- 2002 147 463
- US-A1- 2011 112 576
- US-A1- 2014 296 911
- US-A1- 2019 059 875
- US-A1- 2019 150 912
- US-A1- 2019 343 507

## Description

### CROSS-REFERENCE TO RELATED APPLICATION

The present application claims priority to and the benefit of U.S. Provisional Patent Application Number 63/033,541, filed on June 2, 2020 and entitled "Self Punching Lateral Row Knotless Anchor," U.S. Provisional Patent Application Number 63/033,545, filed on June 2, 2020 and entitled "Internally Fixated Self-Punching Knotless Bone Anchor," and U.S. Provisional Patent Application Number 63/112,389, filed on November 11, 2020 and entitled "Self Punching Lateral Row Assembly and Operation".

### BACKGROUND OF THE INVENTION

### 1. Field of the Invention

The present invention relates to a surgical anchor and anchor driver/deployment device and, more particularly, to a self-punching anchor and driver.

### 2. Description of Related Art

Self-punching bone anchors are commonly used in arthroscopic shoulder repairs because they do not require a pre-punched pilot hole to be implanted. This lets the clinician avoid the potential hardship and hassle of trying to re-find the pre-punched osteotomy in order to insert the anchor, which can be difficult if there is residual soft tissue in the implantation site. Self-punching also removes the implantation error of not implanting the bone anchor along the same longitudinal axis as the pilot hole, which can lead to misalignment and bone anchor fracture. Most of the self-punching bone anchors on the market are variations of a PEEK or resorbable biomaterial screw that pinches the suture to the surrounding bone. However, if the bone quality is poor, and often times it is, the screw cannot find enough purchase in the bone and the implant cannot sufficiently retain the suture or the anchor may even pull out.

Therefore, there is a need for a suture anchor that shifts the suture retention feature internally within the anchor so there is always a strong and consistent pinching of the suture, regardless of bone quality.

The term "suture" as used herein may be any type of filamentous material such as a biocompatible or bioabsorbable filament, ribbon, tape, woven or non-woven material.

Description of the Related Art Section Disclaimer: To the extent that specific patents/publications/products are discussed above in this Description of the Related Art Section or elsewhere in this disclosure, these discussions should not be taken as an admission that the discussed patents/publications/products are prior art for patent law purposes. For example, some or all of the discussed patents/publications/products may not be sufficiently early in time, may not reflect subject matter developed early enough in time and/or may not be sufficiently enabling so as to amount to prior art for patent law purposes.

US 2019/343507 A1 discloses an anchor assembly with the features in the preamble of present claim 1. Other conventional anchors are described in US 2011/112576 A1, US 2019/150912 A1, US 2019/059875 A1, US 2014/296911A1, and US 2002/147463 A1.

### BRIEF SUMMARY OF THE INVENTION

The scope of protection is defined in the appended claims. Embodiments of the present invention are directed to an anchor assembly, self-punching lateral row driver, and self-punching lateral row driver kit. An embodiment of the anchor assembly includes an anchor having a proximal end, a distal self-punching tip, and one or more apertures extending through the proximal end. The assembly also includes a first suture passing aperture extending through the anchor between the proximal end and the distal self-punching tip and a screw configured to engage or abut the proximal end of the anchor.

An embodiment of the self-punching lateral row driver includes a handle assembly including a strike surface and retention cleat, a suture cleat, and a handle body. The handle body is rotatable relative to the suture cleat. The driver also includes a driver tube assembly extending from the suture cleat and an anchor assembly. The anchor assembly includes a proximal screw and a distal anchor with a self-punching tip. The anchor assembly is connected to the driver tube assembly. In a pre-deployment configuration, the screw and the anchor are spaced along the driver tube assembly, and in a post-deployment configuration the screw abuts or engages (or can be positioned at least partially within, or over) the anchor (or can still be spaced from the anchor, just at a distance that is less than the spacing between he proximal screw and the distal anchor in the pre-deployment position). The rotation of the handle moves the screw from the pre-deployment configuration to the post-deployment configuration.

An embodiment of the self-punching lateral row driver kit includes a driver and a suture loader. The driver includes a handle assembly including a strike surface and retention cleat, a suture cleat, and a handle body. The handle body is rotatable relative to the suture cleat. The driver also includes a driver tube assembly extending from the suture cleat and an anchor assembly. The anchor assembly includes a proximal screw and a distal anchor with a self-punching tip. The anchor assembly is connected to the driver tube assembly. The driver also includes a suture passing aperture extending through the anchor. In a pre-deployment configuration, the screw and the anchor are spaced along the driver tube assembly, and in a post-deployment configuration the screw abuts or engages the anchor. The rotation of the handle moves the screw from the pre-deployment configuration to the post-deployment configuration. The suture loader is configured to removably extend through the suture passing aperture.

These and other aspects of the invention will be apparent from and elucidated with reference to the embodiment(s) described hereinafter.

### BRIEF DESCRIPTION OF THE SEVERAL VIEWS OF THE DRAWING(S)

The present invention will be more fully understood and appreciated by reading the following Detailed Description in conjunction with the accompanying drawings. The accompanying drawings illustrate only typical embodiments of the disclosed subject matter and are therefore not to be considered limiting of its scope, for the disclosed subject matter may admit to other equally effective embodiments. Reference is now made briefly to the accompanying drawings, in which:
FIG. 1A is a front view of a self-punching lateral row driver, according to an embodiment;
FIG. 1B is a perspective view of the distal end of the driver, according to an embodiment;
FIG. 2 is a front view of a self-punching lateral row driver kit, according to an embodiment;
FIG. 3 is a sectioned front view of the driver, according to an embodiment;
FIG. 4A is a front view of the inner driver tube of the driver tube assembly, according to an embodiment;
FIG. 4B is a front view of the inner driver tube connected to the strike surface and retention cleat, according to an embodiment;
FIG. 4C is a front view of the outer driver tube over the inner driver tube, according to an embodiment;
FIG. 5A is a front view of a molded half body piece of the handle body, according to an embodiment;
FIG. 5B is a perspective and front view of the suture cleat and stabilizing yolk, according to an embodiment;
FIG. 5C is a front view of the strike surface and retention cleat, stabilizing yolk, and suture cleat connected within the molded half body piece, according to an embodiment;
FIG. 5D is a front view of the handle assembly, according to an embodiment;
FIG. 6A is a front view of the driver (with the anchor assembly omitted) in the pre-deployment configuration, according to an embodiment;
FIG. 6B is a close-up, front view of anchor and the distal end of the inner driver tube, according to an embodiment;
FIG. 7A is a sectioned front view of the driver in the pre-deployment configuration, according to an embodiment;
FIG. 7B is a top view of the anchor, according to an embodiment;
FIG. 8A is a front view of the self-punching lateral row driver kit, according to an embodiment;
FIG. 8B is a close-up, front view of the distal end of the driver and the suture loader, according to an embodiment;
FIG. 8C is a perspective view of the distal end of the driver and the suture loader, according to an embodiment;
FIG. 9A is a front view of the driver in the pre-deployment configuration, according to an embodiment;
FIG. 9B is a front view of the driver in the post-deployment configuration, according to an embodiment;
FIG. 9C is a close-up, front view of the anchor assembly in the post-deployment configuration, according to an embodiment;
FIG. 9D is a close-up, side view of the anchor assembly in the post-deployment configuration, according to an embodiment;
FIG. 10A is a front view of a self-punching lateral row driver, according to an alternative embodiment;
FIG. 10B is a side view of the driver, according to an alternative embodiment;
FIG. 10C is a close-up, front view of the distal end of the driver, according to an alternative embodiment;
FIG. 10D is a sectioned front view of the distal end of the driver, according to an alternative embodiment;
FIG. 11 is a front view of the deployment mechanism, torsional mechanism, trigger mechanism, driver tube assembly, and actuator connected within the molded half body piece, according to an embodiment;
FIG. 12A is a close-up, front view of the external threads of the screw, according to an alternative embodiment;
FIG. 12B is a close-up, front view of the external threads of a standard threadform;
FIG. 13A is a close-up, front view of the anchor assembly with locking suture, according to an alternative embodiment;
FIG. 13B is a close-up and internal front view of the anchor assembly with locking suture, according to an alternative embodiment;
FIG. 14A is a close-up, front view of the anchor assembly with locking suture, according to another alternative embodiment;
FIG. 14B is a close-up, back view of the anchor assembly, according to another alternative embodiment;
FIG. 14C is a close-up and sectioned front view of the anchor assembly, according to another alternative embodiment;
FIG. 14D is a close-up front view of the internal and external threads of the anchor assembly, according to another alternative embodiment;
FIG. 14E is a close-up, front view of the anchor assembly, according to yet another alternative embodiment;
FIG. 14F is a close-up, sectioned front view of the anchor assembly, according to yet another alternative embodiment;
FIG. 14G is a close-up, back view of the anchor assembly with locking suture, according to yet another alternative embodiment;
FIG. 14H is a close-up, sectioned back view of the anchor assembly, according to yet another alternative embodiment;
FIG. 15A is a close-up, front view of the anchor assembly in the pre-deployment configuration, according to an altemative embodiment;
FIG. 15B is a close-up, sectioned front view of the anchor assembly in the pre-deployment configuration, according to an alternative embodiment;
FIG. 15C is a close-up, sectioned front view of the anchor assembly during deployment, according to an alternative embodiment;
FIG. 15D is a close-up, sectioned front view of the anchor assembly in the post-deployment configuration, according to an alternative embodiment;
FIG. 16A is a perspective view of a driver, according to another alternative embodiment;
FIG. 16B is a close-up, perspective view of the distal end of the driver, according to another alternative embodiment;
FIG. 16C is a perspective view of the driver in the pre-deployment configuration at a desired surgical location, according to an embodiment;
FIG. 16D is a perspective view of the driver during deployment, according to an embodiment;
FIG. 16E is a perspective view of the driver in the post-deployment configuration, according to an embodiment;
FIG. 17A is a perspective view of the anchor, according to an alternative embodiment;
FIG. 17B is a top view of the anchor, according to an alternative embodiment;
FIG. 17C is a side view of the anchor, according to an alternative embodiment; and
FIG. 17D is a sectioned, front view of the anchor, according to an alternative embodiment.

### DETAILED DESCRIPTION OF THE INVENTION

Aspects of the present invention and certain features, advantages, and details thereof, are explained more fully below with reference to the non-limiting examples illustrated in the accompanying drawings. Descriptions of well-known structures are omitted so as not to unnecessarily obscure the invention in detail. It should be understood, however, that the detailed description and the specific non-limiting examples, while indicating aspects of the invention, are given by way of illustration only, and are not by way of limitation. Various substitutions, modifications, additions, and/or arrangements, within the scope of the underlying inventive concepts will be apparent to those skilled in the art from this disclosure.

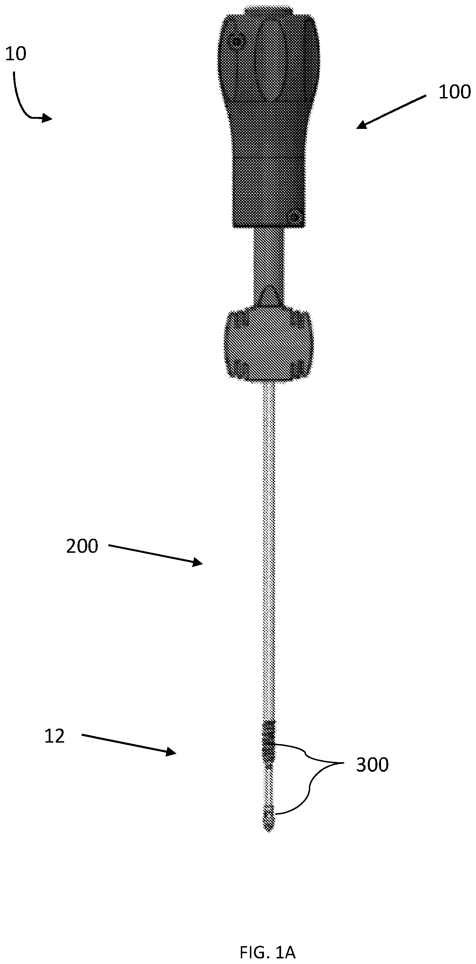

Referring now to the figures, wherein like reference numerals refer to like parts throughout, FIG. 1A is a front view of self-punching lateral row driver (hereafter "driver") 10 in its assembled state, according to an embodiment. The driver 10 comprises a proximal handle assembly 100 extending to a driver tube assembly 200. The driver tube assembly 200 extends to an anchor assembly 300 at a distal end 12 of the driver 10. Portions of the anchor assembly 300 can be made from one or more of the following compositions/materials PEEK, glass fiber and a polymer such as PLA, PGA or PCL; however, the anchor assembly 300 can be made of metal or another polymer strong enough to punch a pilot hole into bone. Having an all-PEEK solution, for example, can be more biocompatible than a solution with metal components left in the patient.

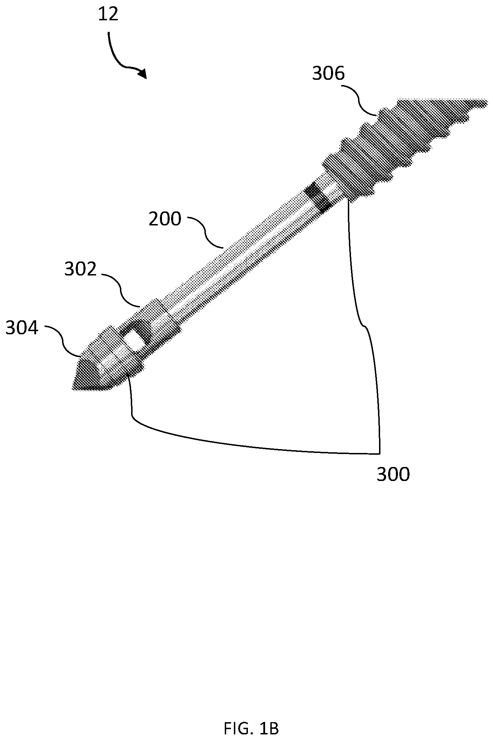

Referring now to FIG. 1B, there is shown a perspective view of the distal end 12 of the driver 10, according to an embodiment. As previously mentioned, the distal end 12 comprises the anchor assembly 300. The anchor assembly 300 is connected to the driver tube assembly 200 and comprises an anchor 302 with a distally positioned self-punching tip 304. The anchor assembly 300 also comprises a screw 306 (proximally positioned with respect to the anchor 302) connected to the driver tube assembly 200. The distal self-punching tip can be made from a material (see, e.g., previous paragraph) that is the same as or different from the proximal non-self-punching portion of the anchor 302. Portions of or the anchor as a whole can be made from the same and/or different material as compared to the screw 306. In the pre-deployment configuration shown in FIGs. 1A-1B, the screw 306 and the anchor 302 are spaced apart from each other along the driver tube assembly 200.

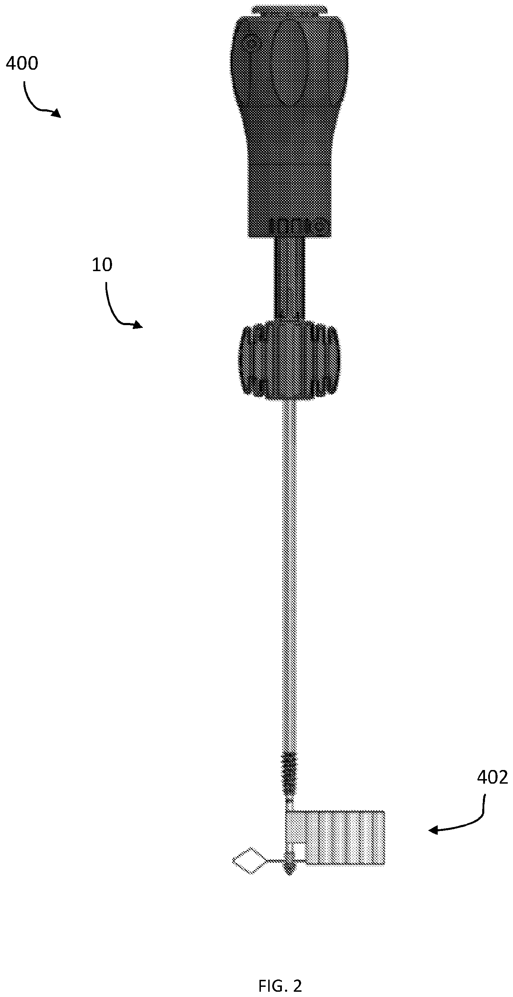

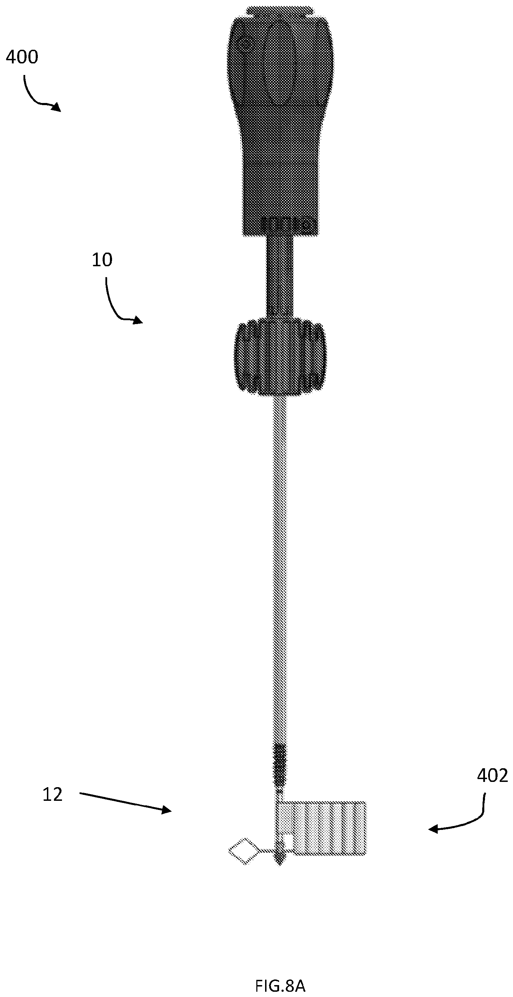

Turning briefly to FIG. 2, there is a front view of a self-punching lateral row driver kit (hereafter "kit") 400. The kit 400 includes the driver 10 of FIGs. 1A and 1B and a suture loader 402. The suture loader 402 is described in detail below with reference to FIGs. 8A-8C.

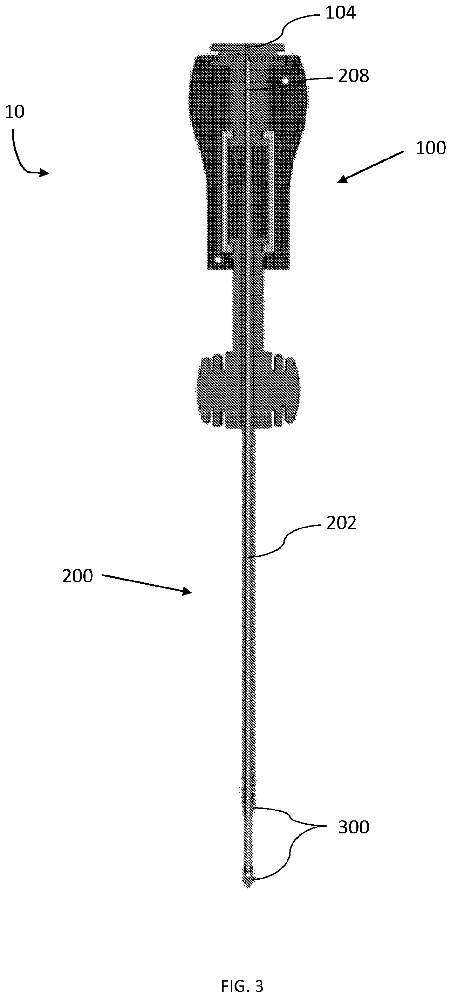

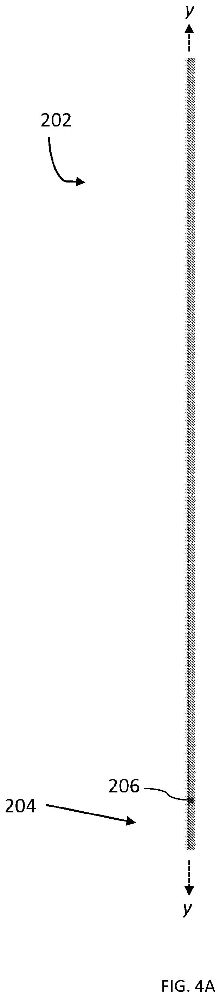

Referring now to FIG. 3, there is a sectioned front view of the driver 10, according to an embodiment. FIG. 3 shows the connections of the components of the driver 10. The driver tube assembly 200 extends through the handle assembly 100 and connects to the anchor assembly 300. Aspects of the handle assembly 100 and driver tube assembly 200 are shown in FIGs. 4A-4C. The driver tube assembly 200 comprises an inner driver tube 202. The inner driver tube 202 is cannulated. At a distal end 204 of the inner driver tube 202, there is an indicator 206. In FIGs. 4A-4C, the indicator 206 extends across the inner driver tube 202, i.e., perpendicular to the central axis y - y extending through the inner driver tube 202. In use, the indicator 206 allows the user to see when the driver 10 has reached the desired depth at the desired surgical location.

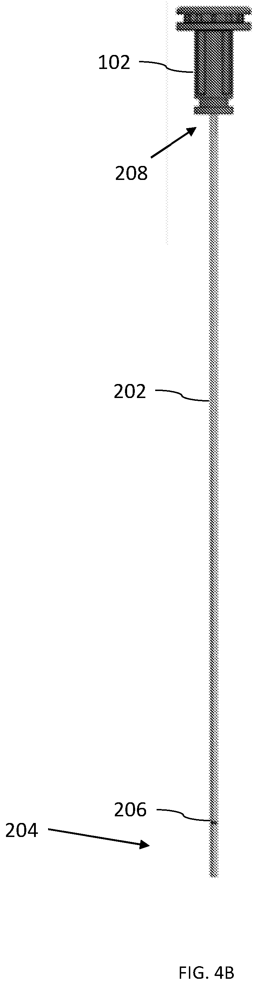

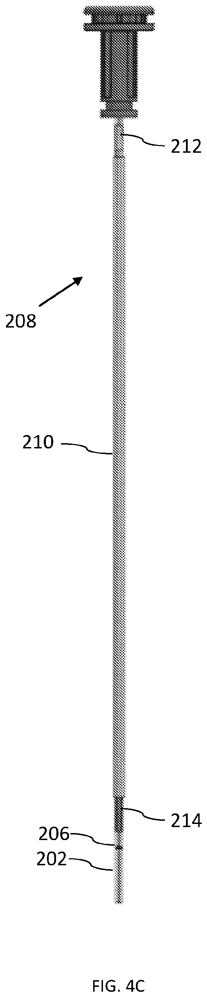

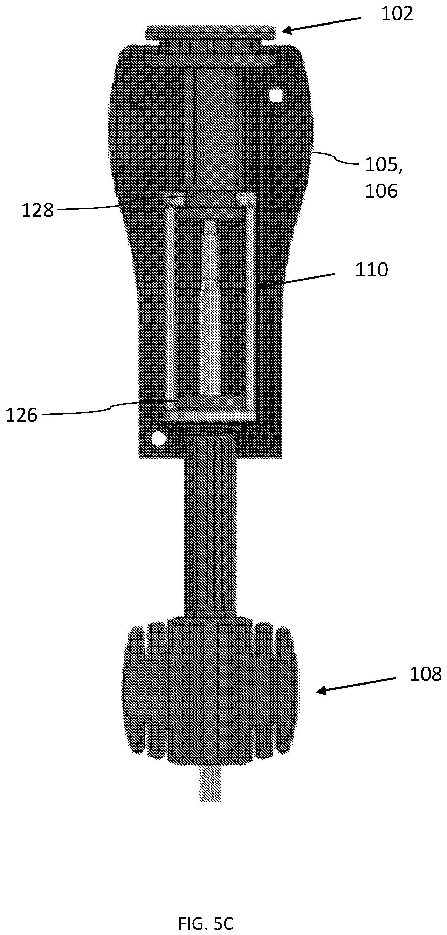

As shown in FIG. 4B, the handle assembly 100 comprises a strike surface and retention cleat 102 connected to a proximal end 208 of the inner driver tube 202. FIG. 3 shows the proximal end 208 of the inner driver tube 202 extending through a channel 104 in the strike surface and retention cleat 102. The driver tube assembly 200 additionally includes a concentric hex (but is not limited to being so shaped) outer driver tube 210, as shown in FIG. 4C. The outer driver tube 210 is cannulated to accommodate the inner driver tube 202 therein. The outer driver tube 210 has a proximal hex feature 212 and a distal hex feature 214. In FIG. 4C, the distal hex feature 214 is proximal relative to the indicator 206 in the pre-deployment configuration. The proximal hex feature 212 can mate with a complimentary feature in the handle (see FIG. 5C), so that when the handle is turned the outer driver tube can turn with it and assist with driving the screw 306 (which mates with the distal hex feature 214) into a bone hole formed by the anchor 302 (as described below).

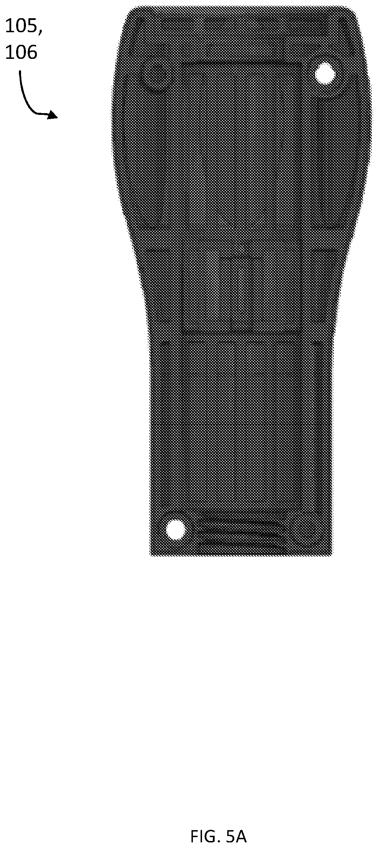

Referring now to FIGs. 5A-5D, there are shown various views of the handle assembly 100 of the driver 10, according to an embodiment. In the depicted embodiment, the handle assembly 100 is composed of a handle body 105 formed by two molded half body pieces 106. Note, although only one of the half pieces 106 is shown in FIG. 5A, the other half body piece 106 is a compatible mirrored version.

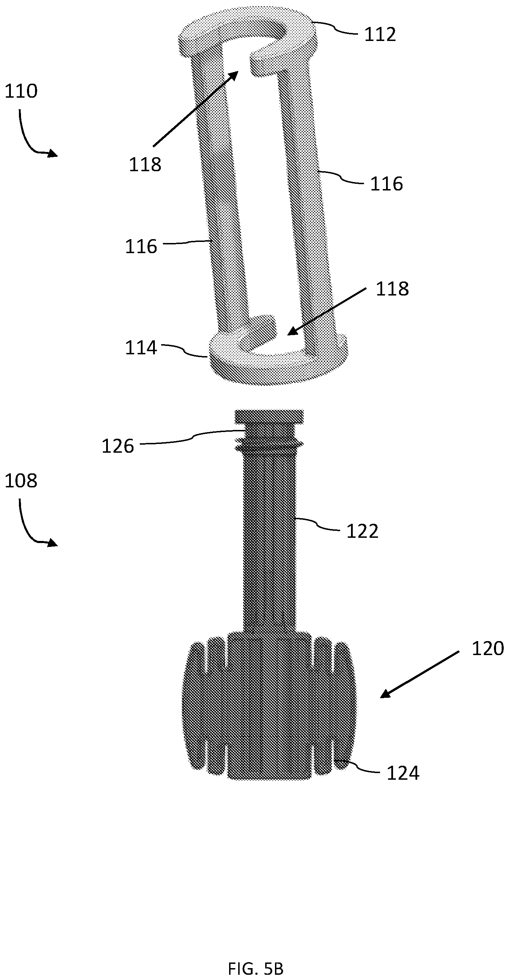

FIG. 5B shows a suture cleat 108 and a stabilizing yolk 110 of the handle assembly 100. The stabilizing yolk 110 is tubular with a proximal ring 112 and a distal ring 114 connected by two rods 116. The proximal and distal rings 112, 114 each have openings 118. The opening 118 in the proximal ring 112 extends therein in an opposite direction as the opening 118 in the distal ring 114, as shown in FIG. 5B. The suture cleat 108 is generally T-shaped having a cleat portion 120 with a connecting rod 122 extending proximally therefrom. The cleat portion 120 has one or more channels 124 extending partially therethrough. In the embodiment shown in FIG. 5B, there are eight channels 124 arranged such that four channels 124 oppose and are aligned with the remaining four channels 124 in pairs.

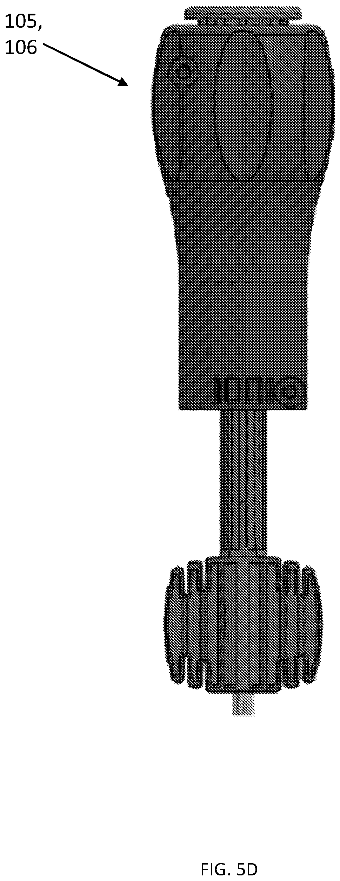

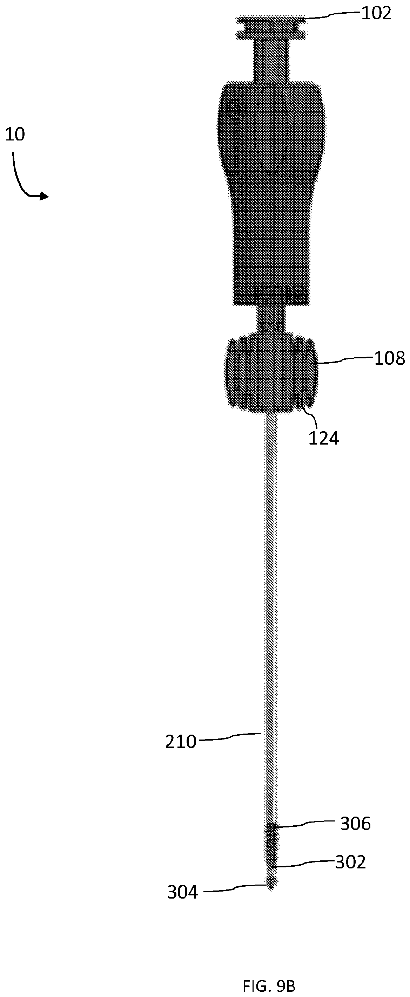

FIG. 5C shows the suture cleat 108, the stabilizing yolk 110, and the strike surface and retention cleat 102 connected within the handle body 105 of the handle assembly 100. As shown, the opening 118 in the distal ring 114 (FIG. 5B) receives and connects to a proximal end 126 of the connecting rod 122 of the suture cleat 108. The opening 118 (FIG. 5B) in the proximal ring 112 receives and connects to a distal end 128 of the strike surface and retention cleat 102. The suture cleat 108, the stabilizing yolk 110, and the strike surface and retention cleat 102 are placed within one of the molded half body pieces 106 of the handle body 105. As shown in FIG. 5D, the remaining molded half body piece 106 of the handle body 105 is attached to first molded half body piece 106, creating the enclosed handle body 105. The handle body 105 and the attached driver tube 210 can move with respect to the suture cleat 108, the stabilizing yolk 110, and the strike surface and retention cleat 102. When the handle 105 moves in a distal direction (downward, as shown in the drawings and described below, with the outer driver tube 210), the suture cleat 108, the stabilizing yolk 110, and the strike surface and retention cleat 102 slide in the opposite direction within a space in the interior of the handle 105 from a pre-deployment position and configuration shown in FIG. 9A to a post-deployment position and configuration shown in FIG. 9B.

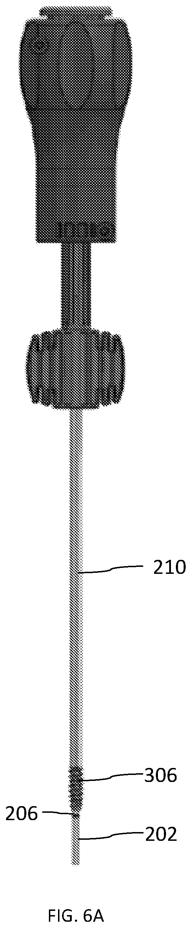

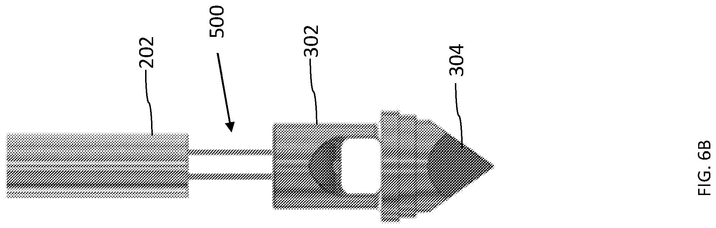

In the pre-deployment configuration, the screw 306 is added to the driver tube assembly 200, as shown in FIGs. 1A-1B. Specifically, as shown in FIG. 6A, the screw 306 is cannulated, has an internal hex shape that mates and slides onto the distal hex feature 214 (FIG. 4C) of the outer driver tube 210, which aids in deployment of the screw 306 into the bone hole created by the anchor 302 as described below (when the outer tube rotates per rotation of the handle, the outer tube rotates screw over the inner driver tube and drives the screw into the bone hole formed by the anchor 302). In the pre-deployment configuration shown, the screw 306 is proximal relative to the indicator 206 of the inner driver tube 202. The inner driver tube 202 connects to the anchor 302 having the distal self-punching tip 304, as shown in FIG. 6B. The inner driver tube 202 is secured to the anchor assembly 300 with a retention suture 500 (which can be pulled out and discarded post-insertion of anchor assembly 300).

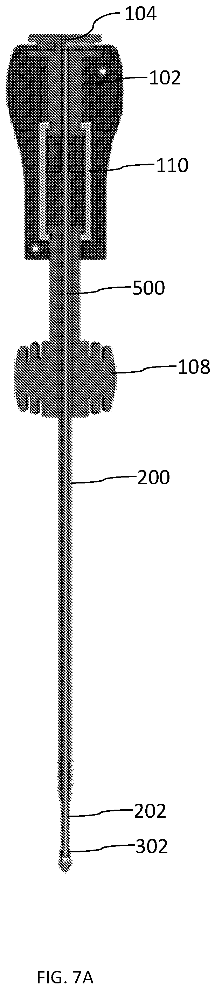

Referring now to FIG. 7A, there is shown a sectioned front view of the driver 10 in the pre-deployment configuration, according to an embodiment. As recited above, retention suture 500 connects the anchor 302 to the driver tube assembly 200. In FIG. 7A, the retention suture 500 extends from the anchor 302 through the cannulated inner driver tube 202, through the suture cleat 108, and through the stabilizing yolk 110. The retention suture 500 then extends from the stabilizing yolk 110, through the channel 104 in the strike surface and retention cleat 102. To maintain the tension keeping the anchor 302 connected to the inner driver tube 202, the retention suture 500 is cleated or otherwise secured around the strike surface and retention cleat 102, as shown in FIG. 7A. In the depicted embodiment, the retention suture 500 is wrapped around a diameter of the strike surface and retention cleat 102.

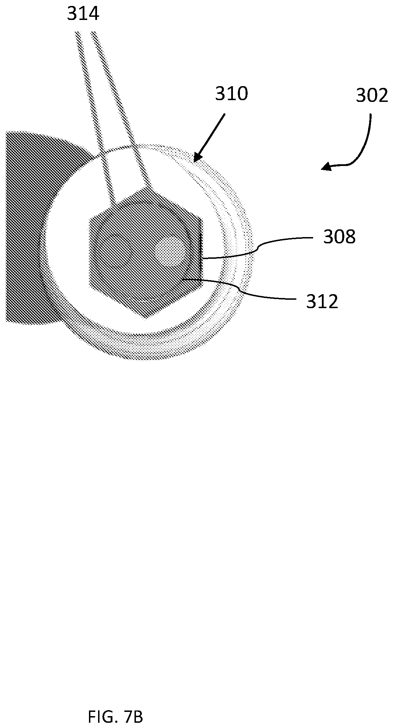

Turning to FIG. 7B, there is shown a top view of the anchor 302 looking through the screw 306 in a distal direction, according to an embodiment. The screw 306 comprises a hex receiving feature 308 in its proximal end 310 that is sized and configured to engage the distal hex feature 214 of the outer driver tube 210. The proximal end of the anchor 302 comprises a recessed area with a surface 312 (which can be circular) sized and configured to receive the inner driver tube 202 (the inner tube 202 can have a diameter that is less than the diameter of the recessed area). The surface 312 extends in a plane perpendicular to the longitudinal axis of the anchor 302, which comprises at least two apertures 314 to secure the retention suture 500 (in an alternative embodiments not forming part of the invention, the surface with the at least two apertures can be positioned at the very proximal end of the anchor 302, which can include no recessed area). Specifically, in the embodiment shown in FIG. 7B, the surface 312 comprises two apertures 314 that receive the retention suture 500 to secure the anchor 302 to the strike surface and retention cleat 102. In use, the retention suture 500 is passed through one of the apertures 314 and then the other aperture 314, and the free ends of the retention suture 500 are cleated at the strike surface and retention cleat 102, as shown in FIG. 7A.

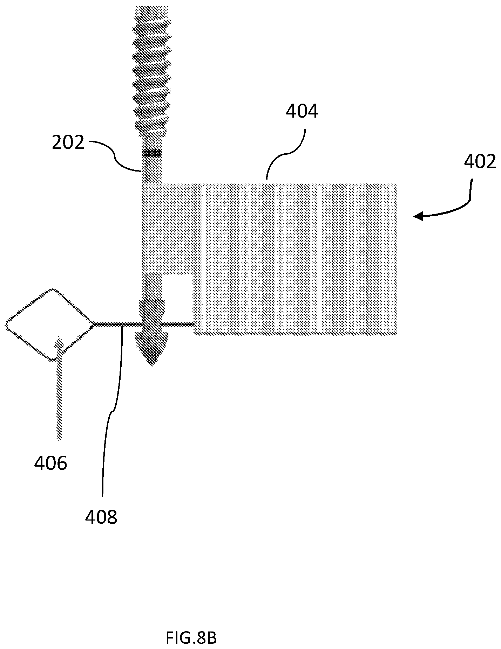

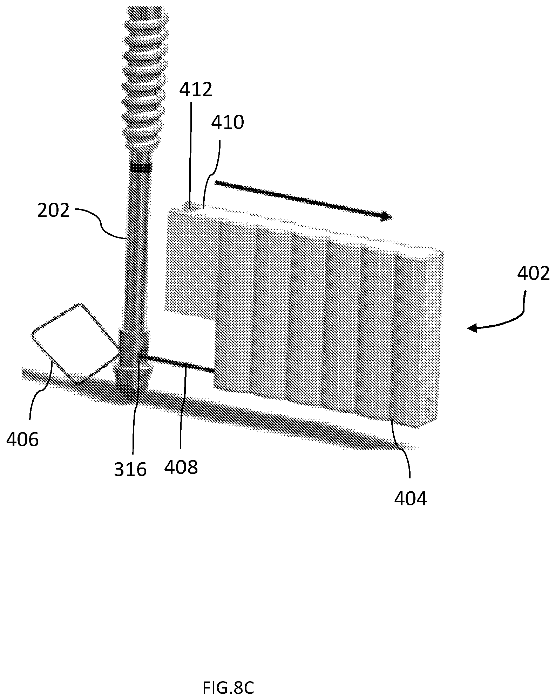

Referring now to FIG. 8A, there is shown a front view of the kit 400, according to an embodiment. The kit 400 includes the driver 10 with the suture loader 402 connected to or otherwise engaged with the distal end 12 of the driver 10. In FIGs. 8B and 8C, the suture loader 402 is shown having a rectangular body 404 with an eyelet 406 extending therefrom. The eyelet 406 can be composed of flexible material, such as nitinol, or other suitable material as should be understood by a person of ordinary skill in the art. In the depicted embodiment, the eyelet 406 is a diamond-shaped when expanded and connects to the rectangular body 402 by a straight portion 408. The rectangular body 404 additionally has a molded portion 410 extending therefrom. As shown in FIG. 8C, the molded portion 410 has a channel 412 extending therethrough that is sized and configured to accommodate the inner driver tube 202. When the eyelet 406 extends through a suture passing aperture 316 in the anchor 302, the channel 412 in the molded portion 410 receives the inner driver tube 202, as shown in FIG. 8B. The inner driver tube 202 may rest within the channel 412 or the molded portion 410 may form a snap fit around the inner driver tube 202.

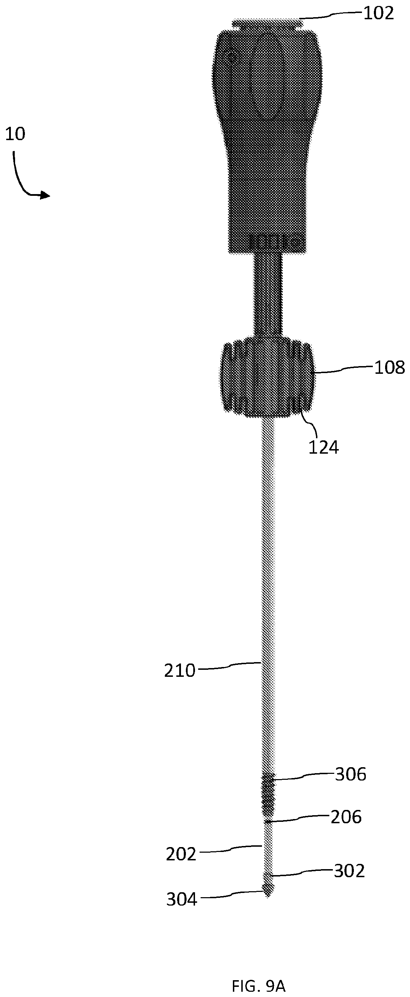

Turning to FIG. 9A, there is shown a front view of the driver 10 in the pre-deployment configuration, according to an embodiment. In the pre-deployment configuration, the suture loader 402 (FIGs. 8A-8C) is used to load locking suture (not shown) onto the anchor 302. As should be understood by a person of ordinary skill in the art in conjunction with a review of this disclosure, the locking suture can be connected at an end to soft tissue that is used in part of a repair procedure. To load the locking suture on the anchor 302, the eyelet 406 of the suture loader 402 is first passed through the suture passing aperture 316 in the anchor 302. A free end of the locking suture is threaded through the eyelet 406 and the eyelet 406 is then passed back through the suture passing aperture 316 in the anchor 302, threading the locking suture through the anchor 302. The suture loader 402 is then removed, leaving the locking suture extending through the anchor 302, as should be understood by a person of ordinary skill in the art in conjunction with a review of this disclosure.

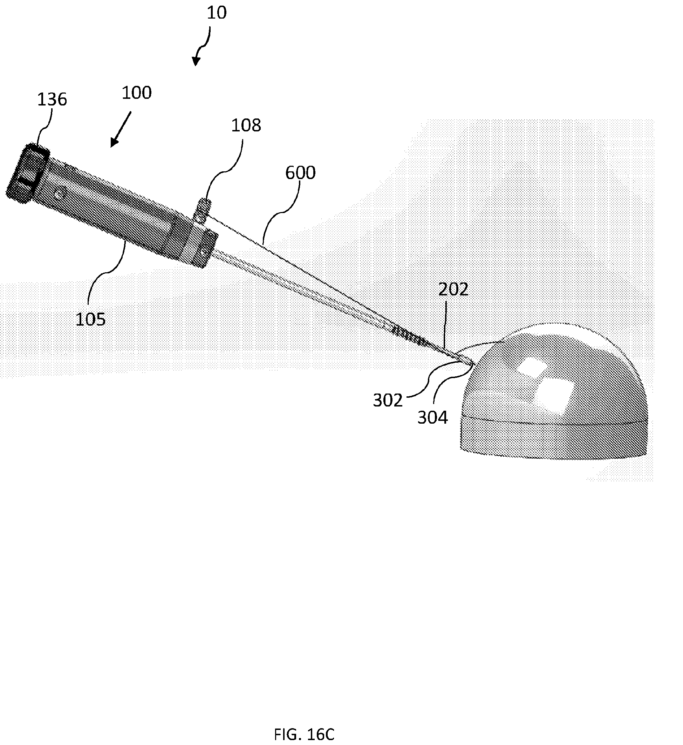

Thereafter, the self-punching tip 304 is positioned at the desired surgical location near the soft tissue in need of repair, not shown (as should be understood by a person of ordinary skill in the art in conjunction with a review of this disclosure; see generally FIGS. 16C-E for a similar illustrative example series of steps in conjunction with bone hole formation and insertion with respect to an alternative embodiment described herein). The locking suture (not shown) connected to soft tissue is then secured to the suture cleat 108. Specifically, the locking suture is wrapped through the channels 124 in the suture cleat 108 to maintain the tension in the locking suture. The locking suture secured around the suture cleat 108 can be adjusted and re-secured at any time as the self-punching tip 304 is aligned at the desired surgical location to maintain proper tension in the locking suture.

After the self-punching tip 304 is at the desired surgical location, the strike surface and retention cleat 102 is malleted, using the self-punching tip 304 to insert the anchor 302 into the bone (not shown). The strike surface and retention cleat 102 is malleted until the indicator 206 on the inner driver tube 202 is below the bone surface. Thus, practically, the user continues to mallet the strike surface and retention cleat 102 until the indicator 206 is no longer visible.

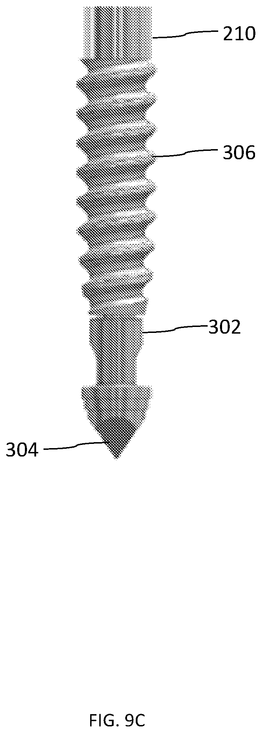

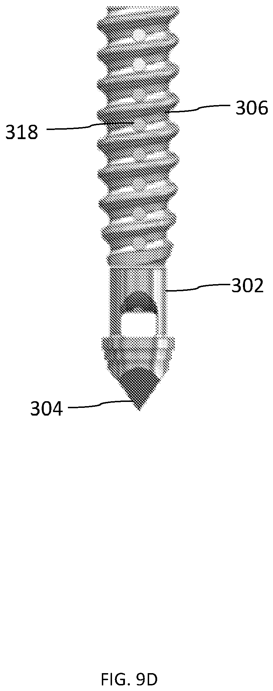

Once the indicator 206 is below the bone surface, the screw 306 is inserted. To insert the screw 306, the user holds the suture cleat 108 steady and rotates the handle body 105 clockwise. The handle body 105 is rotated until the screw 306 is fully inserted, as positionally shown with respect to the device itself in FIG. 9B. When the screw 306 is fully inserted, it abuts or engages the proximal end 310 of the anchor 302. Coupling the punching and implantation of the anchor 302 reduces the time of surgery and removes some of the hassles clinicians face when using an anchor that must have a pilot hole pre-punched. FIG. 9C shows a close-up, front view of the screw 306 and the anchor 302 in the post-deployment configuration, according to an embodiment. As shown, the screw 306, attached to the outer driver tube 210, is connected to or engages the anchor 302, as shown in FIG. 9C. After the screw 306 is inserted, the driver tube assembly 200 and suture 500 can be removed from the surgical location, leaving the screw 306 and the anchor 302. In the close-up, side view shown in FIG. 9D, the screw 306 can additionally comprise one or more vents 318 extending at least partially therethrough. The vents 318 are bone marrow vents that allow bone marrow to grow into and engage the screw 306.

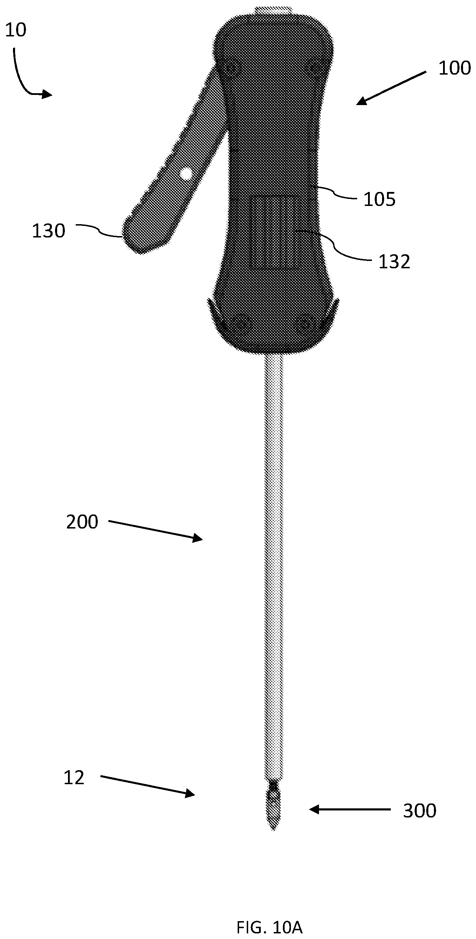

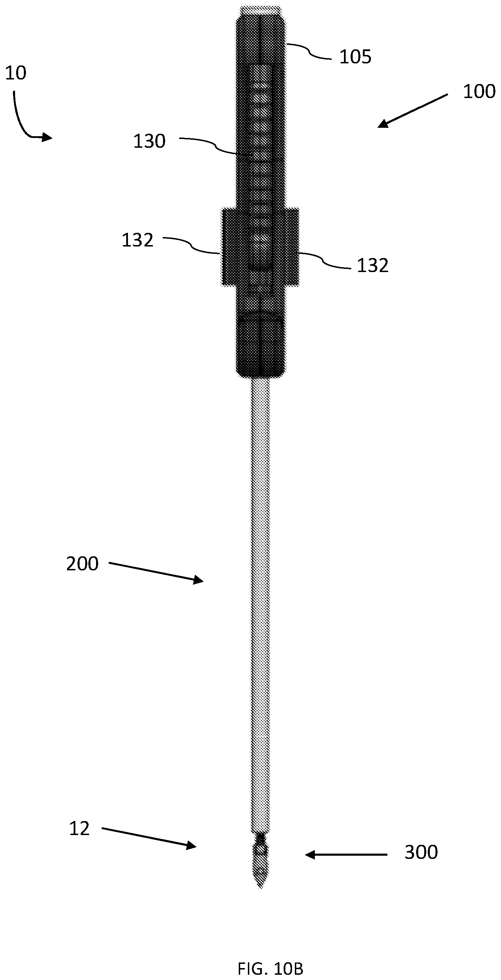

Referring now to FIGs. 10A-13B, there are shown multiple views of an alternative embodiment of the driver 10. FIG. 10A shows a front view of the driver 10, according to an alternative embodiment. The driver 10 comprises a proximal handle assembly 100 extending to a driver tube assembly 200. The driver tube assembly 200 extends to an anchor assembly 300 at a distal end 12 of the driver 10. The handle assembly 100 comprises a handle body 105 with a deployment mechanism 130 extending therefrom. The deployment mechanism 130 is at least partially rotatable relative to the handle body 105. In the side view of the driver 10 shown in FIG. 10B, the handle assembly 100 additionally comprises a torsional mechanism 132.

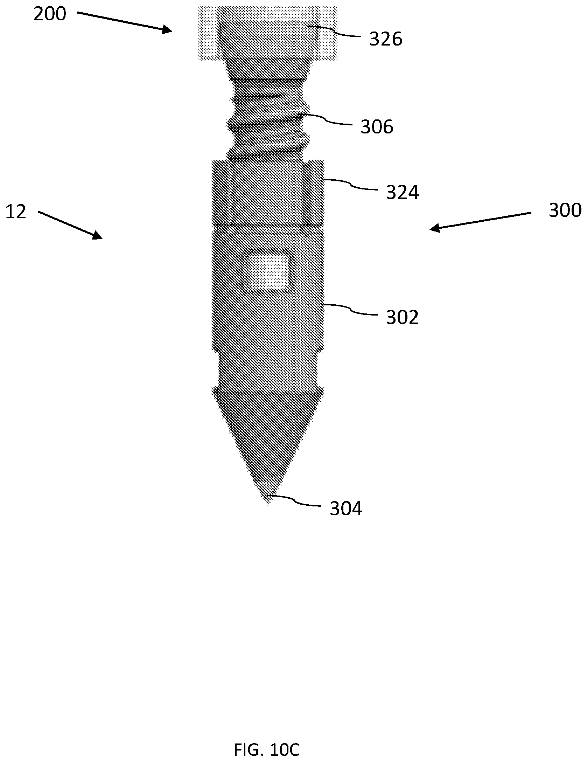

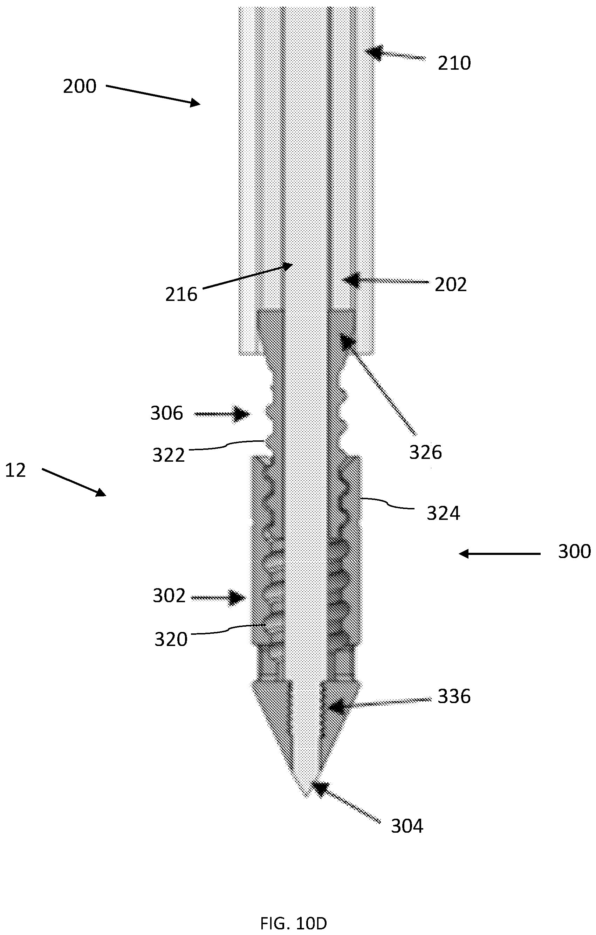

Turning to FIGs. 10C and 10D, there is shown a close-up, front view and a sectioned front view of the distal end 12 of the driver 10, according to an alternative embodiment. As shown in FIG. 10C, the anchor assembly 300 is connected to the driver tube assembly 200. The anchor assembly 300 comprises a screw 306 and an anchor 302. In use, both the anchor 302 and the screw 306 will be implanted into the body of the patient. FIG. 10D shows clearly that the driver tube assembly 200 comprises a cannulated inner driver tube 202 within a cannulated outer driver tube 210. The screw 306 is connected to the inner driver tube 202. As also shown in FIG. 10D, an actuator 216 extends through the inner driver tube 202, screw 306, and anchor assembly 300. In the depicted embodiment, the actuator 216 is a rod with a self-punching tip 304. As shown in FIGs. 10C and 10D, the self-punching tip 304 extends out distally from the anchor 302. The anchor 302 comprises internal threads 320 that are configured to mate with external threads 322 of the screw 306, as shown in FIG. 10D.

As shown in FIG. 10C, the anchor 302 additionally comprises one or more wing features 324 created by strategic cuts or channels in a proximal end 310 of the anchor 302. The wing features 324 are designed to expand and deploy into the surrounding bone as a proximal end 326 of the screw 306 is advanced via the torsional mechanism 132. Thus, the anchor 302 is the main retention feature implanted into the bone. The deploying the of the wing features 324 increases the overall outer dimensions of the anchor 302, making it larger than the osteotomy in which it was implanted, stabilizing the anchor 302 into the bone and resisting pullout.

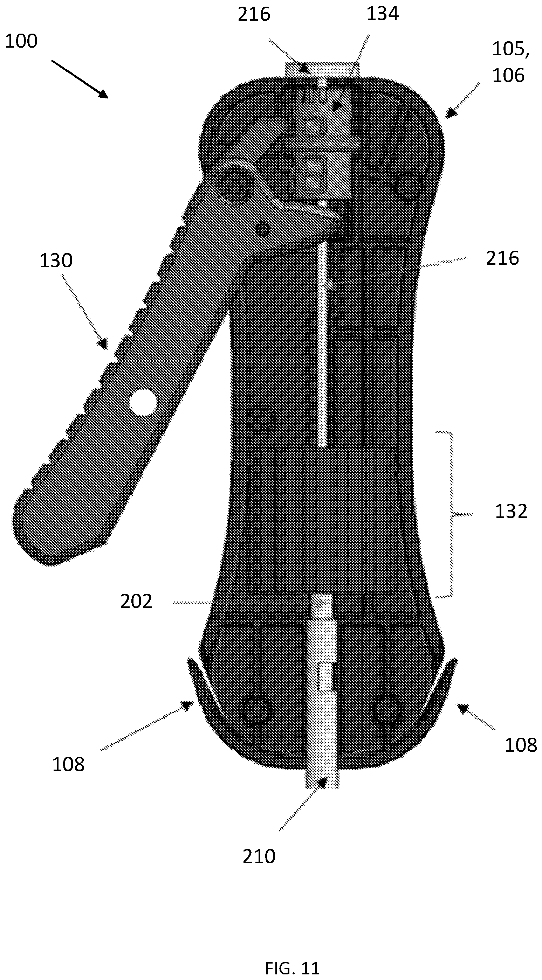

Referring to FIG. 11, there is shown a close-up, front view of the handle assembly 100 (one molded half body piece 106) of the driver 10, according to an alternative embodiment. As shown, the handle body 105 is composed of two molded half body pieces 106 (note, only one is shown in FIG. 11). As shown in FIG. 11, the outer driver tube 210 with the inner driver tube 202 extending at least partially therein is placed within one of the molded half body pieces 106. The outer driver tube 210 protects the inner driver tube 202 and the actuator 216 during implantation and shield them from the surrounding tissue. The inner driver tube 202 is connected to the torsional mechanism 132 and the torsional mechanism 132 is rotatable. Therefore, when the torsional mechanism 132 is rotated, the inner driver tube 202 rotates as well, rotating the connected screw 306. The torsional mechanism 132 is also a screw feature. The torsional mechanism 132 can be a single, dual, or quad lead screw feature. The torsional mechanism 132 is rotated to deploy the screw 306 into the anchor 302.

Still referring to FIG. 11, the actuator 216 extends through the torsional mechanism 132 and a trigger mechanism 134. The trigger mechanism 134 is connected to or selectively engages the deployment mechanism 130. The trigger mechanism 134 is used to pull the actuator 216 and deploy the anchor 302. In use, when the deployment mechanism 130 is engaged (i.e., rotated toward the handle body 105), the trigger mechanism 134 is engaged and pulls the actuator 216 proximally. As shown in FIG. 11, the actuator 216 extends out of the handle body 105. The portion of the actuator 216 that extends out of the handle body 105 is circular or disk-shaped in FIG. 11 and functions as a striking surface for malleting. The actuator 216 drives the anchor 302 into the bone and retains the anchor 302 connection to the handle assembly 100. The handle assembly 100 additionally includes one or more suture cleats 108. In the depicted embodiment, the handle body 105 includes two cleats 108 formed in the molded half body piece 106 and extending proximally. The suture cleat 108 maintains its position to provide consistent, proper tension and placement of locking suture during the use of the torsional mechanism 132.

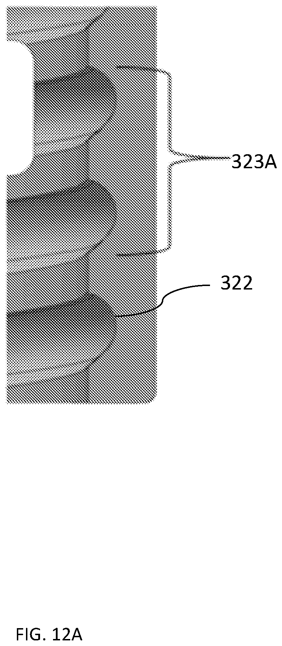

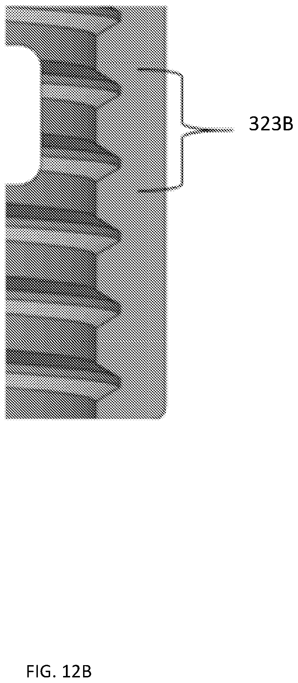

The structure of the anchor assembly 300 maximizes internal suture retention. External threads 322 (i.e., male threadform) of the screw 306 are undersized compared to internal threads 320 (i.e., female threadform) of the anchor 302 to allow room for the locking suture to be compressed between the external threads 322 and the internal threads 320. The threadform used for both the external threads 322 and the internal threads 320 has a wide, rounded profile 323A shown in FIG. 12A instead of a tighter, triangular profile 323B of a standard threadform shown in FIG. 12B. In use, the locking suture is in tension across the opening of the internal threads 320. The wide, rounder profile (FIG. 12A) requires less force to compress the locking suture into the wider and rounder internal threads 320 (FIG. 12A) than the narrower and sharper opening of the standard threadform (FIG. 12B). The standard threadform requires too much force to compress the locking suture therein, and the locking suture may resist the compression and resist the advancement of the screw 306. The rounded threadform shown in FIG. 12A, instead, has a larger pitch, more consistent line-to-line spacing for creating an even spread of the reaction forces of the locking suture from compression, and a larger cross-sectional area to resist deformation as compared to the standard threadform in FIG. 12B.

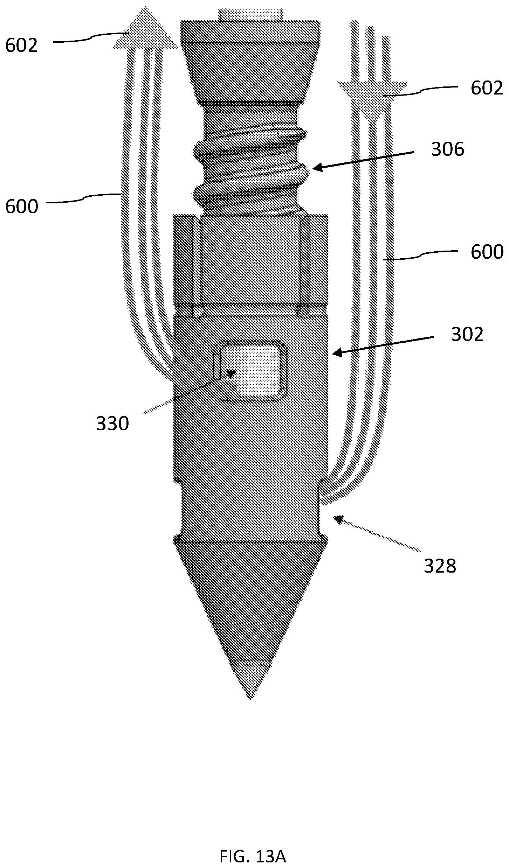

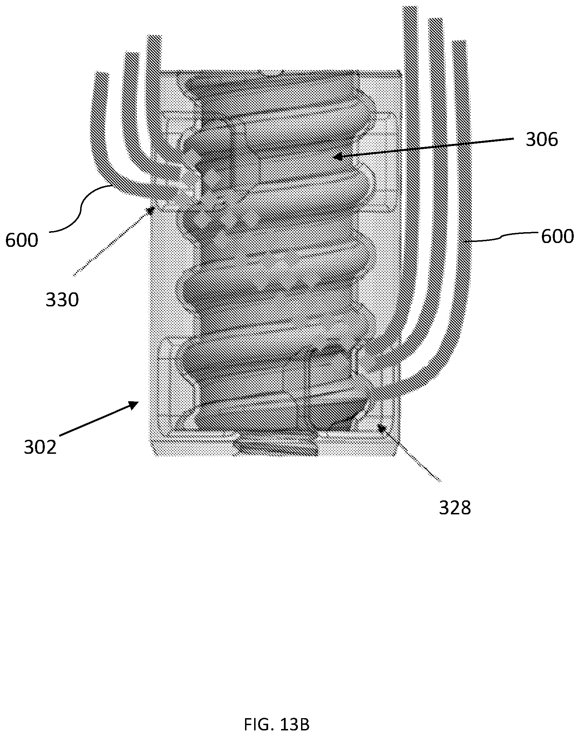

To use the driver 10, the user first loads it with locking suture 600, as shown in FIGs. 13A and 13B. As described in conjunction with the first embodiment described herein, locking suture 600 connected to the soft tissue to be repaired is threaded through the anchor 302. The locking suture 600 may comprise two pre-loaded suture pull tabs 602 with nitinol wire. For loading, the locking suture 600 is passed in through a distal suture passing aperture 328 that extends through the anchor 302, as shown in FIGs. 13A and 13B. The locking suture 600 is passed out through a proximal suture passing aperture 330 that extends through the anchor 302, as also shown in FIGs. 13A and 13B. The apertures 328, 330 are sized and configured such that the anchor 302 can hold a total of six (6) limbs of locking suture, with each pull tab 602 holding a maximum of three (3) limbs of locking suture. The apertures 328, 330 are also offset to maximize the amount of material of the anchor 302 that the locking suture 600 pulls against to maximize the internal compression force, as described above.

Thereafter, the user inserts the driver 10 through a cannula or through the soft tissue of the patient to the desired surgical location. Then, the user punches the driver 10 into the bone with a mallet. In particular, the user can strike the portion of the actuator 216 that extends out proximally from the handle body 105. The actuator 216 is malleted until it reaches an indicated laser mark (e.g., on the inner driver tube 202) to a depth of at least 2 mm below the surface of the bone. The user then tensions the anchor 302 relative to the repair tissue by pulling on the locking suture 600. The locking suture 600 is cleated to the suture cleat 108 of the handle body 105 after the desired tension is reached.

To lock the locking suture 600 in place within the anchor 302, the user rotates the torsional mechanism 132, thereby rotating the connected screw 306 into the anchor 302 and trapping the locking suture 600 between the screw 306 and the anchor 302. This process also deploys the wing features 324. In other words, as the screw 306 moves distally within the anchor 302, the screw 306 forces the wing features 324 outward. Once the screw 306 is completely inserted within the anchor 302, the user deploys the anchor 302 and detach the anchor 302 from the handle assembly 100. The user deploys and detaches the anchor 302 by depressing the deployment mechanism 130 toward the handle body 105. Depressing the deployment mechanism 130 pulls the actuator 216 proximally, which, in turn, pulls the anchor 302 against the driver tube assembly 200 until the force strips the plastic, left-handed internal threads 336 (FIG. 10D) of the anchor 302 and deploys the anchor 302.

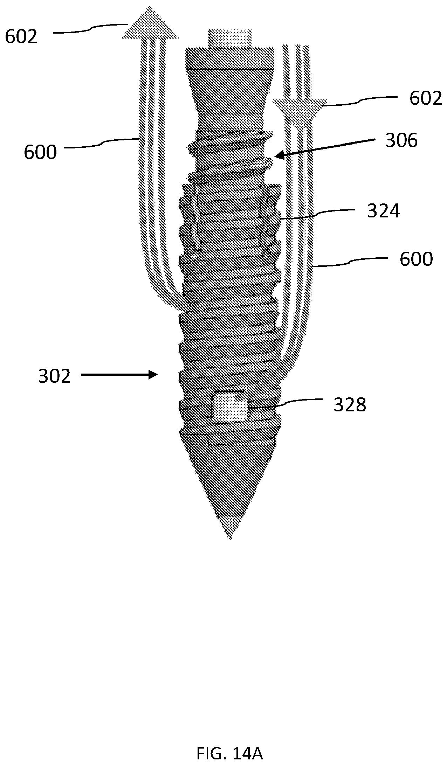

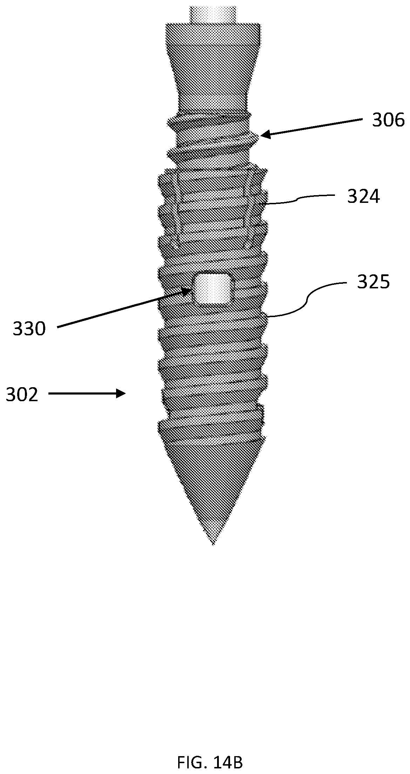

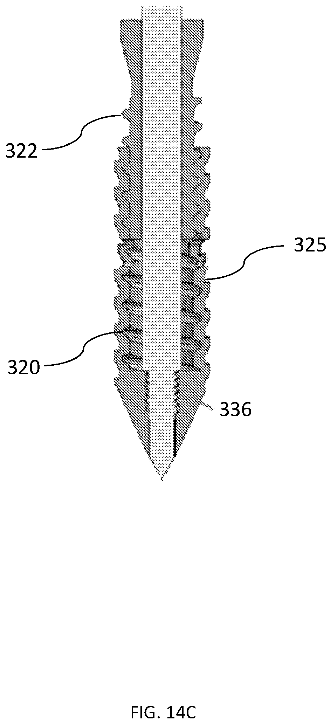

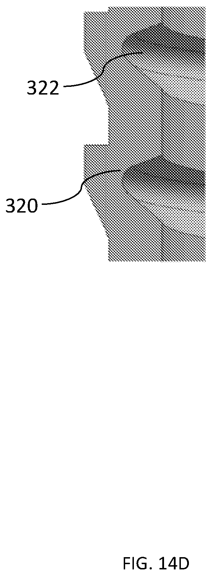

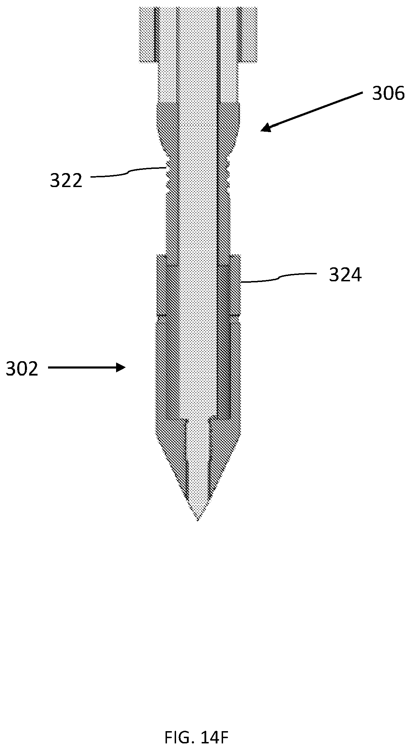

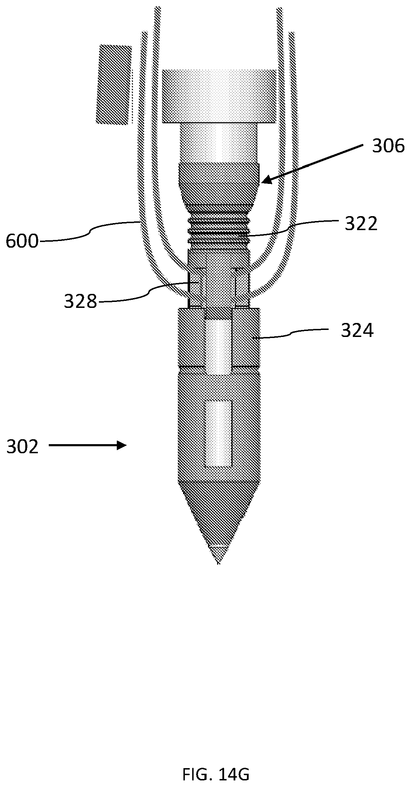

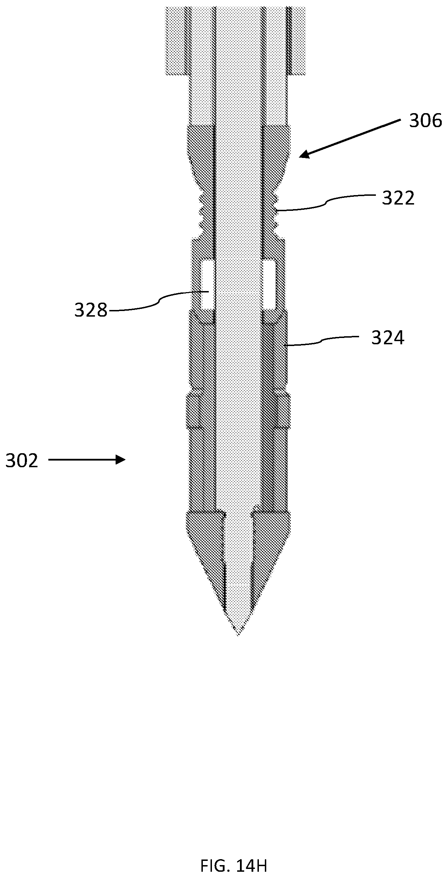

Referring now to FIGs. 14A-14D, there are shown various views of an alternative embodiment of the anchor assembly 300. The anchor assembly 300 shown in FIGs. 14A-14D is similar in function to that shown in FIGs. 13A-13B. The anchor assembly 300 in FIGs. 14A-14D comprises a screw 306 and an anchor 302. The anchor assembly 300 is configured to be malleted into the desired surgical location. The screw 306 has wider, rounded external threads 322 that are configured to mate with wider, rounded internal threads 320 of the anchor 302 (FIG. 14D). As shown in FIGs. 14B-14C, the anchor 302 comprises external threads 325 in the depicted embodiment. The anchor 302 additionally comprises wing features 324 that are configured to pop or move outward (5.46 mm) when the screw 306 is rotated distally into the anchor 302. Furthermore, the anchor 302 comprises a distal suture passing aperture 328 that is offset from a proximal suture passing aperture 330, as described above with reference to FIGs. 13A-13B, which each accommodate at least three (3) threads of locking suture 600. The anchor 302 also comprises plastic, left-handed internal threads 336 (FIG. 14C) to deploy the anchor 302, as described above.

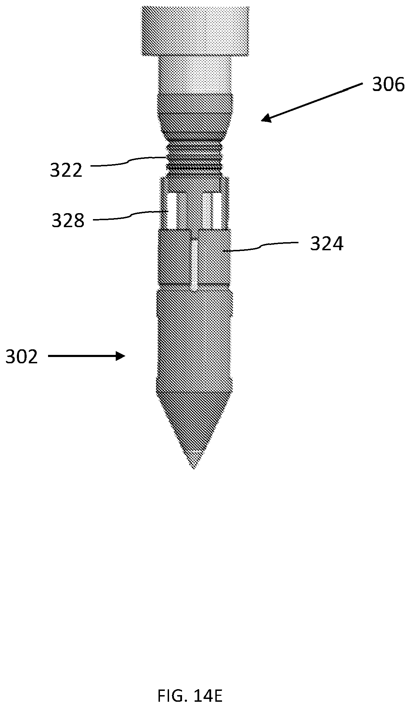

Referring now to FIGs. 14E-14H, there are shown various views of another alternative embodiment of the anchor assembly 300. The anchor assembly 300 in FIGs. 14E-14H uses a "pull" style deployment. The anchor assembly 300 comprises a tubular "punch portion" 306 (instead of a screw) and an anchor 302. The punch portion 306 comprises distal suture passing apertures 328 that are sized and configured to accommodate four (4) limbs of locking suture 600 therethrough. This differs from the previous embodiments in that the punch portion 306 has the distal suture passing apertures 328 instead of the anchor 302. During deployment, the punch portion 306 is guided into the anchor 302 and the locking suture 600 is compressed between the punch portion 306 and the anchor 302. As shown, the punch portion 306 comprises some external threads 322 that assist in compressing the locking suture 600 against the anchor 302. The anchor 302 additionally comprises wing features 324, as described above, that deform or extend outward (5.46 mm) to lock the anchor 302 in place within the bone.

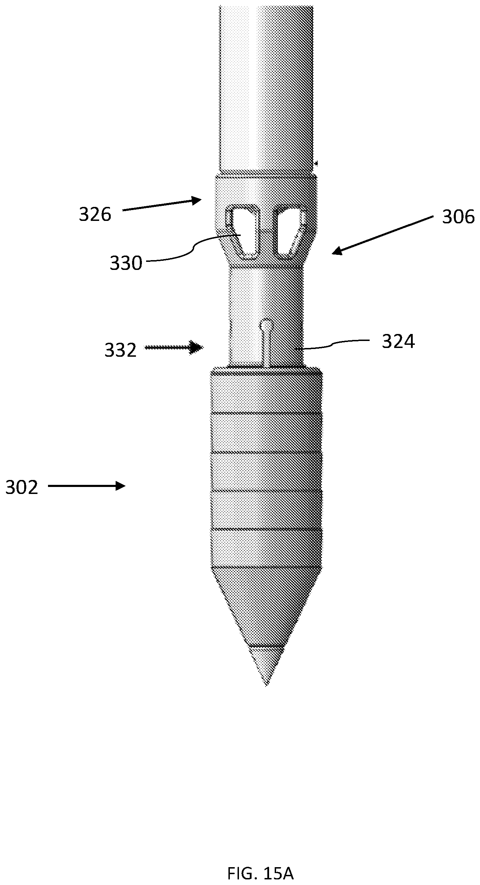

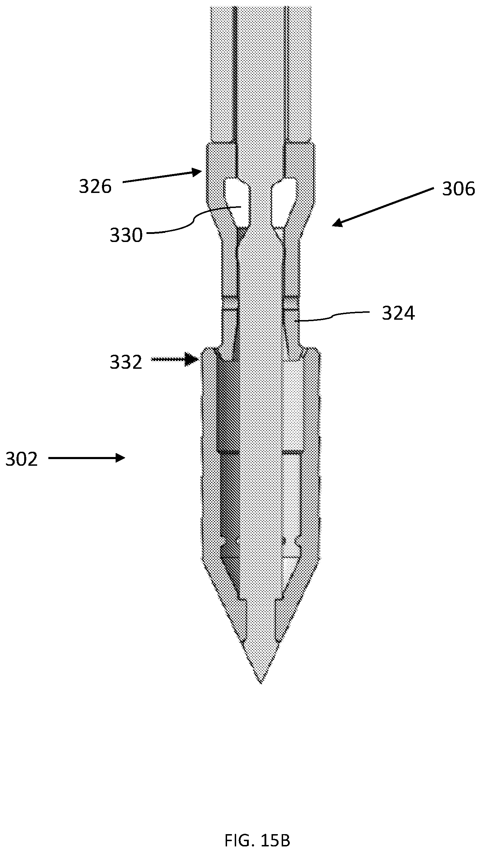

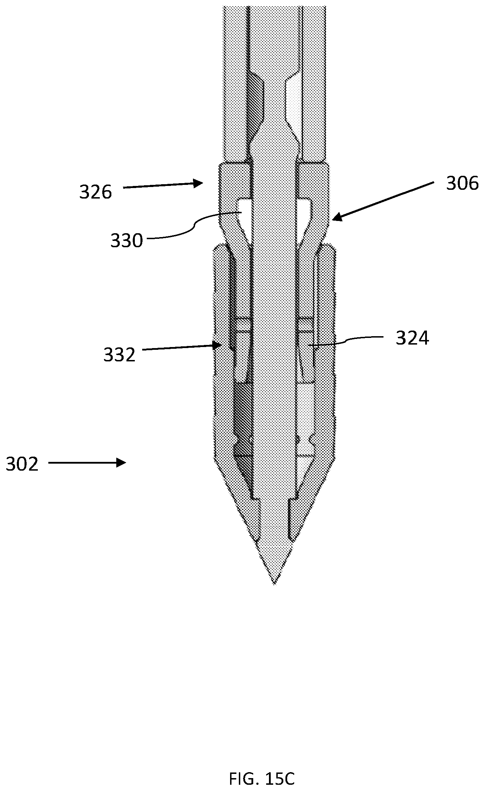

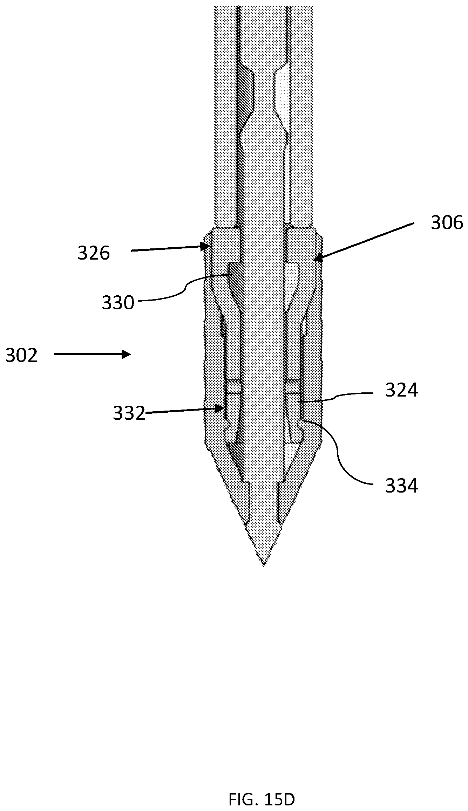

Turning to FIGs. 15A-15D, there are shown various views of yet another alternative embodiment of the anchor assembly 300. The anchor assembly 300 comprises an anchor assembly 300 and a punch portion 306 (again, instead of a screw). The punch portion 306 is configured to be inserted into the anchor 302. The punch portion 306 comprises a proximal end 326 that is wider than a distal end 332. The proximal end 326 comprises proximal suture passing apertures 330 configured to receive locking suture (not shown) therethrough. The distal end 332 of the punch portion 306 comprises wing features 324 that expand or move outward within the anchor 302. As the anchor assembly 300 is deployed, as shown in FIGs. 15C-15D, the punch portion 306 extends into the anchor 302 until the wing features 324 engage a locking feature 334 within the anchor 302. The proximal end 326 of the punch portion 306 may cause a proximal end 310 of the anchor 302 to expand radially when the punch portion 306 is fully deployed within the anchor 302.

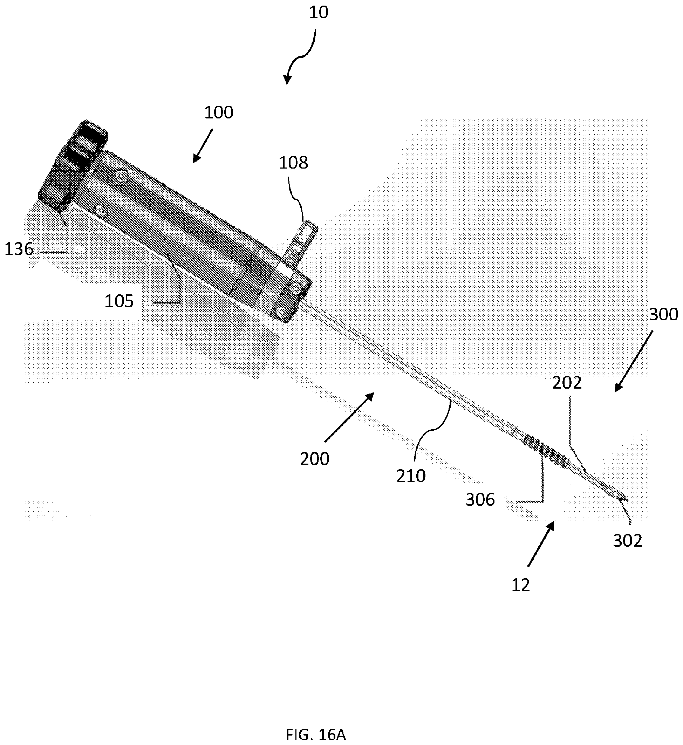

Referring now to FIGs. 16A-16E, there are show various views of another alternative embodiment of the driver 10. As depicted in the perspective view in FIG. 16A, the driver 10 comprises a proximal handle assembly 100 extending to a driver tube assembly 200. The driver tube assembly 200 extends to an anchor assembly 300 at a distal end 12 of the driver 10. The anchor assembly 300 is preferably made of PEEK; however, the anchor assembly 300 can be made of metal or another polymer strong enough to punch a pilot hole into bone.

The handle assembly 100 comprises a proximal, rotatable knob 136 connected to a handle body 105. The handle body 105 comprises a cleat 108 extending therefrom and configured to assist in tensioning suture. The driver tube assembly 200 includes an inner driver tube 202 and an outer driver tube 210. The outer driver tube 210 is cannulated and the inner driver tube 202 extends therein. The inner driver tube 202 is connected to the anchor assembly 300, as described in detail below. The inner driver tube 202 can be fixedly connected to knob 136 and the outer driver tube 210 can be fixedly connected to the handle body 105 or vice versa, depending on which portion of the instrument is driving which portion of the anchor assembly (as should be understood by a person of ordinary skill in the art in conjunction with a review of this disclosure).

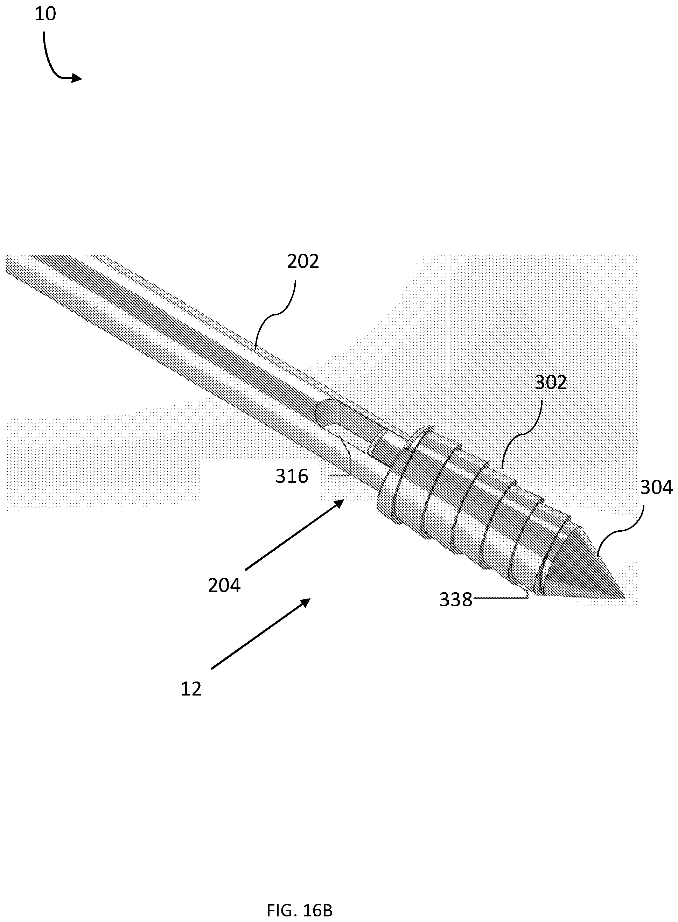

FIG. 16B shows a close-up, perspective view of the distal end 12 of the driver 10. As previously mentioned, the distal end 12 comprises the anchor assembly 300. The anchor assembly 300 is connected to the driver tube assembly 200 and comprises a screw 306 and an anchor 302 with a self-punching tip 304. The anchor 302 comprises a broaching feature 338 extending around and along the length of the anchor 302 that aids in minimizing stress cracking when impacting the self-punching tip 304 into bone. A distal end 204 of the inner driver tube 202 comprises a suture passing aperture 316 extending therethrough.

As shown in FIG. 16A, the screw 306 of the anchor assembly 300 is connected to the driver tube assembly 200. Specifically, the screw 306 is connected to the outer driver tube 210. The screw 306 is proximal relative to the anchor 302 and the suture passing aperture 316. In a pre-deployment configuration (FIG. 16A), the screw 306 and the anchor 302 are spaced along the driver tube assembly 200 with the suture passing aperture 316 therebetween.

FIG. 16C shows a perspective view of the driver 10 in the pre-deployment configuration. In use, locking suture 600 connected to the soft tissue in need of repair is threaded through the suture passing aperture 316 in the inner driver tube 202. The locking suture 600 is then tensioned and wrapped around or otherwise connected to the cleat 108 of the handle body 105, as shown in FIG. 16C. Once tensioned, the self-punching tip 304 of the anchor 302 is aligned at the desired surgical location. Note, the locking suture 600 can be retensioned and cleated again at any time to ensure that the locking suture 600 remains under proper tension.

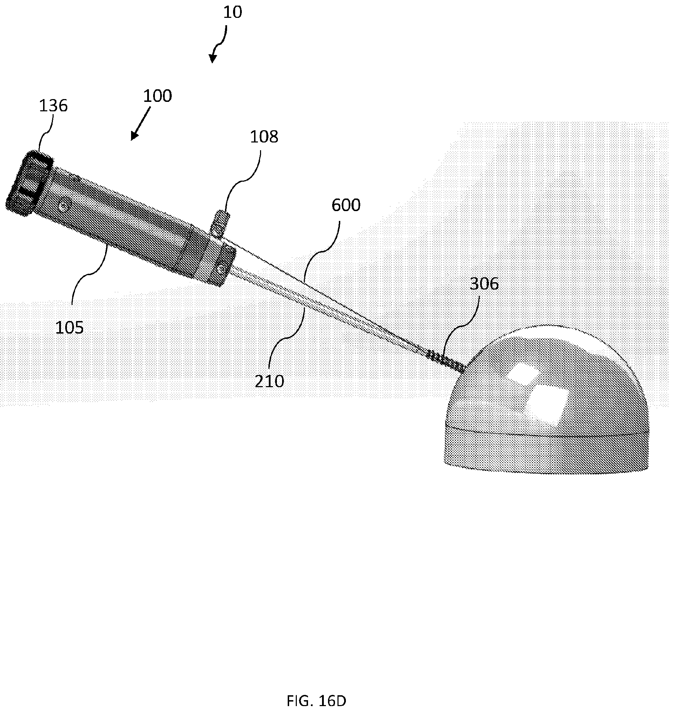

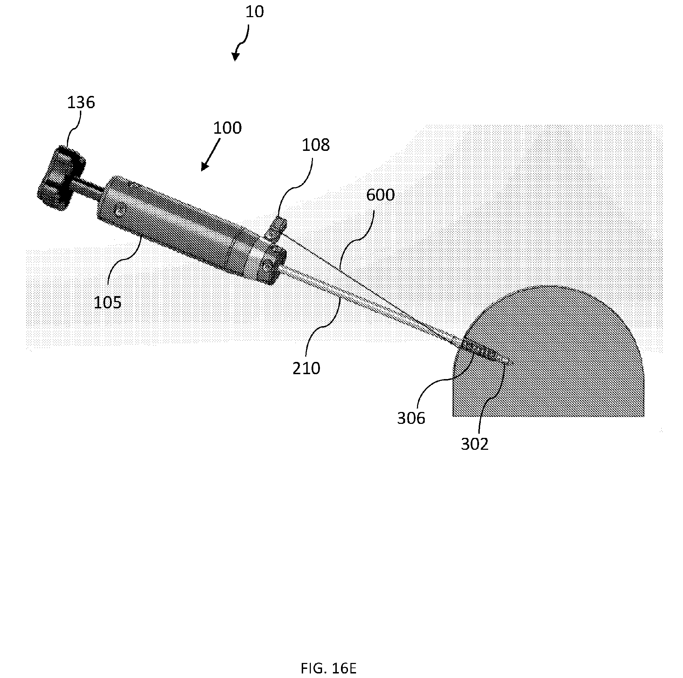

Thereafter, in FIG. 16D, the knob 136 of the handle assembly 100 is malleted or otherwise impacted to punch the self-punching tip 304 into the bone. This is done until the screw 306 comes in contact with the bone surface, as shown in FIG. 16D. From there, the user holds the knob 136 and rotates the handle body 105 relative thereto (or vice versa in an alternative embodiment, depending on which tube 202, 210 is connected to the knob 136 and which is connected to the handle body 105). Rotating the handle body 105 distally toward the desired surgical location inserts the screw 306 into the bone, as shown in FIG. 16E. Because the screw 306 is connected to the outer driver tube 210, which is connected to the handle body 105, when the handle body 105 is rotated, the screw 306 rotates as well. The screw 306 locks the locking suture 600 against the bone. The locking suture 600 also becomes locked between the screw 306 and the anchor 302.

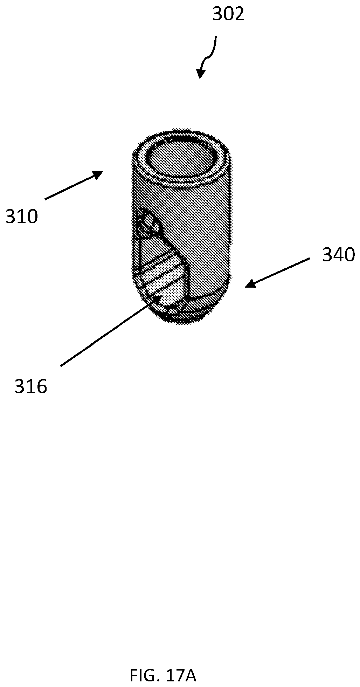

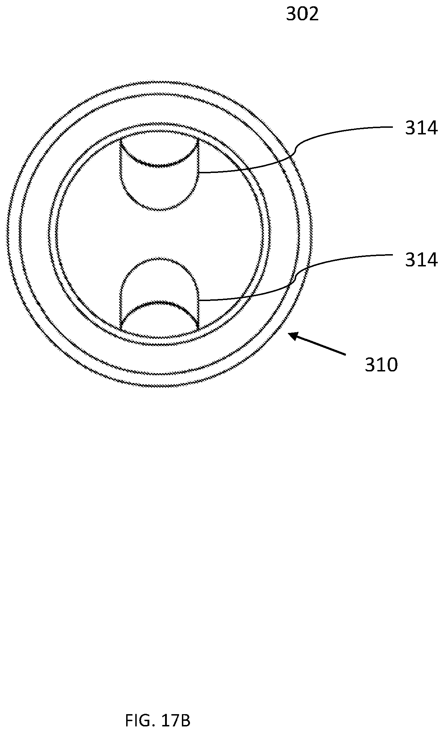

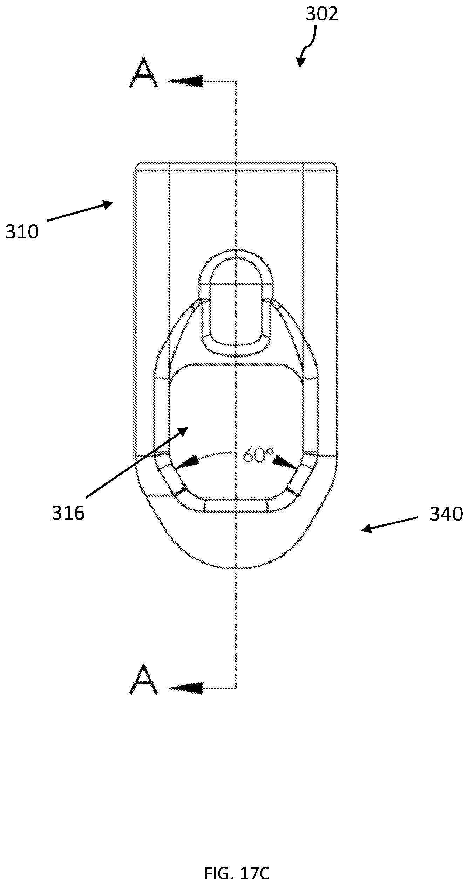

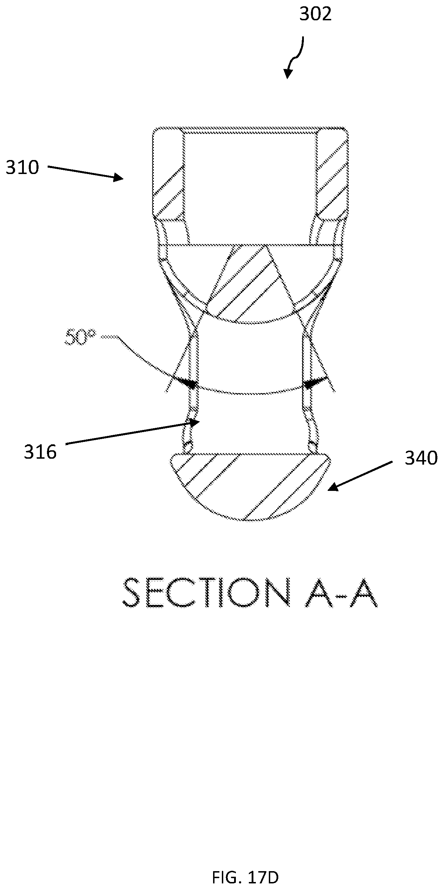

Referring now to FIGs. 17A-17D, there are shown various views of an alternative embodiment of the anchor 302. In the top view in FIG. 17B, the anchor 302 comprises a proximal end 310 with two apertures 314 extending therethrough. The two apertures 314 are sized and configured to receive a retention suture 500 (FIG. 6B) that holds the anchor 302 to a driver tube assembly 200 or handle assembly 100, as described above with reference to FIG. 7B. The anchor 302 also includes a suture passing aperture 316 that is configured to receive locking suture that is connected to the soft tissue to be repaired, as shown in FIG. 7C. FIG. 7D shows that the anchor 302 comprises a wider proximal end 310 as compared to a distal end 340. The wider proximal end 310 allows the anchor 302 to grasp the bone and lock into the pilot hole.

It should be understood that the values used above are only representative values, and other values may be in keeping with the spirit and intention of this disclosure.

While several inventive embodiments have been described and illustrated herein with reference to certain exemplary embodiments, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein (and it will be understood by one skilled in the art that various changes in detail may be effected therein without departing from the scope of the invention as defined by claims that can be supported by the written description and drawings). More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto; inventive embodiments may be practiced otherwise than as specifically described and claimed. Further, where exemplary embodiments are described with reference to a certain number of elements it will be understood that the exemplary embodiments can be practiced utilizing either less than or more than the certain number of elements.

All definitions, as defined and used herein, should be understood to control over dictionary definitions, definitions in documents, and/or ordinary meanings of the defined terms.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. The term "connected" is to be construed as partly or wholly contained within, attached to, or joined together, even if not directly attached to where there is something intervening.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

Approximating language, as used herein throughout the specification and claims, may be applied to modify any quantitative representation that could permissibly vary without resulting in a change in the basic function to which it is related. Accordingly, a value modified by a term or terms, such as "about" and "substantially", are not to be limited to the precise value specified. In at least some instances, the approximating language may correspond to the precision of an instrument for measuring the value. Here and throughout the specification and claims, range limitations may be combined and/or interchanged; such ranges are identified and include all the sub-ranges contained therein unless context or language indicates otherwise.

The recitation of ranges of values herein are merely intended to serve as a shorthand method of referring individually to each separate value falling within the range, unless otherwise indicated herein, and each separate value is incorporated into the specification as if it were individually recited herein.

All methods described herein can be performed in any suitable order unless otherwise indicated herein or otherwise clearly contradicted by context. The use of any and all examples, or exemplary language (e.g., "such as") provided herein, is intended merely to better illuminate embodiments of the invention and does not impose a limitation on the scope of the invention unless otherwise claimed.

No language in the specification should be construed as indicating any non-claimed element as essential to the practice of the invention.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively, as set forth in the United States Patent Office Manual of Patent Examining Procedures, Section 2111.03.

It will be apparent to those skilled in the art that various modifications and variations can be made to the present invention without departing from the scope of the invention. There is no intention to limit the invention to the specific form or forms disclosed, but on the contrary, the intention is to cover all modifications, alternative constructions, and equivalents falling within the scope of the invention, as defined in the appended claims. Thus, it is intended that the present invention cover the modifications and variations of this invention provided they come within the scope of the appended claims and their equivalents.

## Claims

1. An anchor assembly (300), comprising:
a distal anchor (302) extending along a longitudinal axis and having a proximal end (310) and a distal self-punching tip (304);
at least a first aperture (314) extending through the proximal end (310) of the distal anchor (302) in a direction substantially parallel to the longitudinal axis;
a first suture passing aperture (316, 328) extending through the distal anchor (302) at an angle to the longitudinal axis and between the proximal end (310) and the distal self-punching tip (304); and
a proximal screw (306) configured to engage or abut the proximal end (310) of the distal anchor (302),
**characterized in that** the proximal end (310) of the distal anchor (302) includes a recess with a surface (312) positioned therein extending in a plane substantially perpendicular to the longitudinal axis, wherein the at least first aperture (314) and a second aperture (314) are positioned through the surface (312).

2. The anchor assembly (300) of claim 1, further comprising one or more vents (318) extending at least partially through the proximal screw (306).

3. The anchor assembly (300) of claim 1, wherein at least a distal portion of the proximal screw (306) is configured to fit in the recess in the proximal end (310) of the distal anchor (302).

4. The anchor assembly (300) of claim 2, wherein the distal anchor (302) comprises internal threads (320) configured to mate with external threads (322) of the proximal screw (306).

5. The anchor assembly (300) of claim 1, further comprising a second suture passing aperture (330) extending through the distal anchor (302) between the proximal end (310) and the distal self-punching tip (304).

6. The anchor assembly (300) of claim 5, wherein the first suture passing aperture (316, 328) is offset from the second suture passing aperture (330).

7. The anchor assembly (300) of claim 1, wherein the proximal end (310) of the distal anchor (302) comprises one or more wing features (324).

8. The anchor assembly (300) of claim 1, wherein the proximal screw (306) and the distal anchor (302) are made from different compositions or materials.

9. The anchor assembly (300) of claim 1, wherein a proximal end (310) and a distal self-punching tip (304) of the distal anchor (302) are made from different compositions or materials.

10. A self-punching lateral row driver (10), comprising:
a handle assembly (100) comprising a strike surface and a retention cleat (102), a suture cleat (108), and a handle body (105), the handle body (105) rotatable relative to the suture cleat (108);
a driver tube assembly (200) extending from the suture cleat (108);
the anchor assembly (300) of claim 1 connected to the driver tube assembly (200);
wherein in a pre-deployment configuration, the proximal screw (306) and the distal anchor (302) are spaced apart along the driver tube assembly (200) and in a post-deployment configuration, a distal portion of the proximal screw (306) abuts or engages the distal anchor (302); and
wherein the rotation of the handle body (105) moves the screw (306) from the pre-deployment configuration to the post-deployment configuration.

11. The driver (10) of claim 10, wherein the driver assembly (200) includes a cannulated hex outer driver tube (210) with an inner driver tube (202) extending therethrough.

12. The driver of claim 11,
further comprising an indicator (206) extending across the inner driver tube (202), wherein in the pre-deployment configuration, the indicator (206) is preferably positioned between the screw (306) and the anchor (302); and/or
wherein the inner driver tube (202) is connected to the strike surface and retention cleat (102); and/or
wherein the suture cleat (108) is connected to the hex outer driver tube (210).

13. The driver (200) of claim 10, further comprising a retention suture (500) connecting the distal anchor (302) to the strike surface and retention cleat (102).

14. A self-punching lateral row driver kit (400), comprising:
the driver (10) of claim 10; and
a suture loader (402) configured to removably extend through the first suture passing aperture (316, 328).

15. The kit (400) of claim 14, wherein the suture loader (402) comprises a flexible eyelet (406) extending therefrom and, preferably, a molded portion (410) configured to accommodate the driver tube assembly (200) and, more preferably, a rectangular body (404) extending from the molded portion (410) and, most preferably, a locking suture (600) extending through the eyelet (406).

## Patentansprüche

1. Verankerungsanordnung (300), umfassend:
einen distalen Anker (302), der sich entlang einer Längsachse erstreckt und ein proximales Ende (310) und eine distale selbststanzende Spitze (304) aufweist;
mindestens eine erste Öffnung (314), die sich durch das proximale Ende (310) des distalen Ankers (302) in einer Richtung im Wesentlichen parallel zu der Längsachse erstreckt;
eine erste Nahtdurchgangsöffnung (316, 328), die sich durch den distalen Anker (302) in einem Winkel zu der Längsachse und zwischen dem proximalen Ende (310) und der distalen selbststanzenden Spitze (304) erstreckt; und
eine proximale Schraube (306), die dazu konfiguriert ist, in das proximale Ende (310) des distalen Ankers (302) einzugreifen oder daran anzuliegen,
**dadurch gekennzeichnet, dass** das proximale Ende (310) des distalen Ankers (302) eine Ausnehmung mit einer darin angeordneten Fläche (312) aufweist, die sich in einer Ebene im Wesentlichen senkrecht zu der Längsachse erstreckt, wobei die mindestens erste Öffnung (314) und eine zweite Öffnung (314) durch die Fläche (312) hindurch angeordnet sind.

2. Verankerungsanordnung (300) nach Anspruch 1 ferner umfassend eine oder mehrere Entlüftungen (318), die sich zumindest teilweise durch die proximale Schraube (306) erstrecken.

3. Verankerungsanordnung (300) nach Anspruch 1, wobei mindestens ein distaler Abschnitt der proximalen Schraube (306) dazu konfiguriert ist, in die Aussparung in dem proximalen Ende (310) des distalen Ankers (302) zu passen.

4. Verankerungsanordnung (300) nach Anspruch 2, wobei der distale Anker (302) ein Innengewinde (320) umfasst, das dazu konfiguriert ist, mit dem Außengewinde (322) der proximalen Schraube (306) zusammenzupassen.

5. Verankerungsanordnung (300) nach Anspruch 1 ferner umfassend eine zweite Nahtdurchgangsöffnung (330), die sich durch den distalen Anker (302) zwischen dem proximalen Ende (310) und der distalen selbststanzenden Spitze (304) erstreckt.

6. Verankerungsanordnung (300) nach Anspruch 5, wobei die erste Nahtdurchgangsöffnung (316, 328) gegenüber der zweiten Nahtdurchgangsöffnung (330) versetzt ist.

7. Verankerungsanordnung (300) nach Anspruch 1, wobei das proximale Ende (310) des distalen Ankers (302) ein oder mehrere Flügelelemente (324) umfasst.

8. Verankerungsanordnung (300) nach Anspruch 1, wobei die proximale Schraube (306) und der distale Anker (302) aus unterschiedlichen Zusammensetzungen oder Materialien bestehen.

9. Verankerungsanordnung (300) nach Anspruch 1, wobei ein proximales Ende (310) und eine distale selbststanzende Spitze (304) des distalen Ankers (302) aus unterschiedlichen Zusammensetzungen oder Materialien hergestellt sind.

10. Selbststanzender seitlicher Reihenantrieb (10), umfassend:
eine Griffanordnung (100), die eine Schlagfläche und eine Halteklammer (102), eine Nahtklammer (108) und einen Griffkörper (105) aufweist, wobei der Griffkörper (105) relativ zu der Nahtklammer (108) drehbar ist;
eine Antriebsrohranordnung (200), die sich von der Nahtklammer (108) aus erstreckt;
die Verankerungsanordnung (300) nach Anspruch 1, die mit der Antriebsrohranordnung (200) verbunden ist;
wobei in einer Konfiguration vor der Entfaltung die proximale Schraube (306) und der distale Anker (302) einen Abstand entlang der Antriebsrohranordnung (200) voneinander haben und in einer Konfiguration nach der Entfaltung ein distaler Abschnitt der proximalen Schraube (306) an dem distalen Anker (302) anliegt oder mit dieser in Eingriff steht; und
wobei die Drehung des Griffkörpers (105) die Schraube (306) von der Konfiguration vor der Entfaltung in die Konfiguration nach der Entfaltung bewegt.

11. Antrieb (10) nach Anspruch 10, wobei die Antriebsanordnung (200) ein kanüliertes, sechseckiges äußeres Antriebsrohr (210) mit einem sich durch dieses hindurch erstreckenden inneren Antriebsrohr (202) umfasst.

12. Antrieb nach Anspruch 11,
ferner umfassend einen Indikator (206), der sich über das innere Antriebsrohr (202) erstreckt, wobei der Indikator (206) in der Konfiguration vor der Entfaltung vorzugsweise zwischen der Schraube (306) und dem Anker (302) positioniert ist; und/oder
wobei das innere Antriebsrohr (202) mit der Anschlagfläche und der Halteklammer (102) verbunden ist; und/oder
wobei die Nahtklammer (108) mit dem sechseckigen äußeren Antriebsrohr (210) verbunden ist.

13. Antrieb (200) nach Anspruch 10 ferner mit einer Rückhaltenaht (500), die den distalen Anker (302) mit der Schlagfläche und der Halteklammer (102) verbindet.

14. Bausatz (400) für einen selbststanzenden Seitenreihenantrieb, umfassend:
den Antrieb (10) nach Anspruch 10; und
einen Nahtmaterial-Lader (402), der dazu konfiguriert ist, sich entfernbar durch die erste Nahtmaterial-Durchgangsöffnung (316, 328) zu erstrecken.

15. Bausatz (400) nach Anspruch 14, wobei der Nahtmaterial-Lader (402) eine flexible Öse (406) umfasst, die sich von ihm aus erstreckt, und vorzugsweise einen geformten Abschnitt (410), der dazu konfiguriert ist, die Antriebsrohranordnung (200) aufzunehmen, und noch bevorzugter einen rechteckigen Körper (404), der sich von dem geformten Abschnitt (410) aus erstreckt, und am meisten bevorzugt einen Verriegelungsfaden (600), der sich durch die Öse (406) erstreckt.

## Revendications

1. Ensemble (300) d'ancrage, comprenant :
un ancrage distal (302) s'étendant le long d'un axe longitudinal et ayant une extrémité proximale (310) et une pointe distale auto-perforante (304) ;
au moins une première ouverture (314) s'étendant à travers l'extrémité proximale (310) de l'ancrage distal (302) dans un sens sensiblement parallèle à l'axe longitudinal ;
une première ouverture (316, 328) de passage de suture s'étendant à travers l'ancrage distal (302) à un angle par rapport à l'axe longitudinal et entre l'extrémité proximale (310) et la pointe distale auto-perforante (304) ; et
une vis proximale (306) configurée pour s'engager avec ou buter contre l'extrémité proximale (310) de l'ancrage distal (302),
**caractérisé en ce que** l'extrémité proximale (310) de l'ancrage distal (302) inclut un renfoncement avec une surface (312) positionnée dans celui-ci s'étendant dans un plan sensiblement perpendiculaire à l'axe longitudinal, dans lequel l'au moins une première ouverture (314) et une deuxième ouverture (314) sont positionnées à travers la surface (312).

2. Ensemble (300) d'ancrage selon la revendication 1, comprenant en outre une ou plusieurs lumières (318) s'étendant au moins partiellement à travers la vis proximale (306).

3. Ensemble (300) d'ancrage selon la revendication 1, dans lequel au moins une partie distale de la vis proximale (306) est configurée pour s'ajuster dans le renfoncement dans l'extrémité proximale (310) de l'ancrage distal (302).

4. Ensemble (300) d'ancrage selon la revendication 2, dans lequel l'ancrage distal (302) comprend un filetage interne (320) configuré pour correspondre avec un filetage externe (322) de la vis proximale (306).

5. Ensemble (300) d'ancrage selon la revendication 1, comprenant en outre une deuxième ouverture (330) de passage de suture s'étendant à travers l'ancrage distal (302) entre l'extrémité proximale (310) et la pointe distale auto-perforante (304).

6. Ensemble (300) d'ancrage selon la revendication 5, dans lequel la première ouverture (316, 328) de passage de suture est décalée par rapport à la deuxième ouverture (330) de passage de suture.

7. Ensemble (300) d'ancrage selon la revendication 1, dans lequel l'extrémité proximale (310) de l'ancrage distal (302) comprend une ou plusieurs caractéristiques d'ailettes (324).

8. Ensemble (300) d'ancrage selon la revendication 1, dans lequel la vis proximale (306) et l'ancrage distal (302) sont constitués de compositions ou matériaux différent(e)s.

9. Ensemble (300) d'ancrage selon la revendication 1, dans lequel une extrémité proximale (310) et une pointe distale auto-perforante (304) de l'ancrage distal (302) sont constitués de compositions ou matériaux différent(e)s.

10. Dispositif d'entraînement (10) à rangée latérale auto-perforant, comprenant :
un ensemble (100) de poignée comprenant une surface de frappe et un crampon (102) de retenue, un crampon (108) de suture, et un corps (105) de poignée, le corps (105) de poignée rotatif par rapport au crampon (108) de suture ;
un ensemble (200) de tubes de dispositif d'entraînement s'étendant depuis le crampon (108) de suture ;
l'ensemble (300) d'ancrage selon la revendication 1 connecté à l'ensemble (200) de tubes de dispositif d'entraînement ;
dans lequel, dans une configuration pré-déploiement, la vis proximale (306) et l'ancrage distal (302) sont espacés le long de l'ensemble (200) de tubes de dispositif d'entraînement et dans une configuration post-déploiement, une partie distale de la vis proximale (306) bute contre ou s'engage avec l'ancrage distal (302) ; et
dans lequel la rotation du corps (105) de poignée déplace la vis (306) de la configuration pré-déploiement à la configuration post-déploiement.

11. Dispositif d'entraînement (10) selon la revendication 10, dans lequel l'ensemble (200) de tubes de dispositif d'entraînement inclut un tube extérieur hexagonal cannelé (210) de dispositif d'entraînement avec un tube intérieur (202) de dispositif d'entraînement s'étendant à travers celui-ci.

12. Dispositif d'entraînement selon la revendication 11,
comprenant en outre un indicateur (206) s'étendant à travers le tube intérieur (202) de dispositif d'entraînement, dans lequel, dans la configuration pré-déploiement, l'indicateur (206) est préférablement positionné entre la vis (306) et l'ancrage (302) ; et/ou
dans lequel le tube intérieur (202) de dispositif d'entraînement est connecté à la surface de frappe et au crampon (102) de retenue ; et/ou
dans lequel le crampon (108) de suture est connecté au tube extérieur hexagonal (210) de dispositif d'entraînement.

13. Dispositif (200) d'entraînement selon la revendication 10, comprenant en outre une suture (500) de retenue connectant l'ancrage distal (302) à la surface de frappe et au crampon (102) de retenue.

14. Kit (400) de dispositif d'entraînement à rangée latérale auto-perforant, comprenant :
le dispositif (10) d'entraînement selon la revendication 10 ; et
un chargeur (402) de suture configuré pour s'étendre de façon amovible à travers la première ouverture (316, 328) de passage de suture.

15. Kit (400) selon la revendication 14, dans lequel le chargeur (402) de suture comprend un œillet flexible (406) s'étendant depuis celui-ci et, préférablement, une partie moulée (410) configurée pour recevoir l'ensemble (200) de tubes de dispositif d'entraînement et, plus préférablement, un corps rectangulaire (404) s'étendant depuis la partie moulée (410) et, le plus préférablement, une suture (600) de verrouillage s'étendant à travers l'œillet (406).
